# EUROPEAN PATENT SPECIFICATION

(11) **EP 1 265 626 B1**
(45) Date of publication and mention of the grant of the patent: **10.09.2014**
(21) Application number: 01922399.9
(22) Date of filing: 14.03.2001
(51) Int. Cl.: A61K 38/00, A61K 38/02, A61K 38/23, A61K 39/00, A61K 39/395, A61P 11/00

(54) **METHOD FOR REDUCING ALLERGEN-INDUCED AIRWAY HYPERRESPONSIVENESS**
METHODE ZUR REDUZIERUNG ALLERGEN-INDUZIERTER LUFTWEGS-HYPERREAKTIVITÄT
METHODE DE DIMINUTION D'UNE HYPERSENSIBILITE DES VOIES RESPIRATOIRES INDUITE PAR DES ALLERGENES

(30) Priority: 14.03.2000 US 189622 P
(43) Date of publication of application: 18.12.2002
(73) Proprietor: National Jewish Health, Denver, CO 80206 (US)
(72) Inventor: GELFAND, Erwin, W., Denver, CO 80110 (US); DAKHAMA, Azzedine, Aurora, CO 80014 (US)
(74) Representative: Evens, Paul Jonathan
(86) International application number: PCT/US2001/008264
(87) International publication number: WO 2001/068118

(56) References cited:
- US-A- 5 635 478
- US-A- 5 858 978

## Description

### FIELD OF THE INVENTION

This invention relates to compositions to reduce allergen-induced airway hyperresponsiveness, by activating or increasing the activity of a CGRP receptor in the lungs of the mammal. The invention also describes a method of identifying compounds useful for reducing allergen-induced airway hyperresponsiveness in a mammal.

### BACKGROUND OF THE INVENTION

Sensory neuropeptides play an important role in the pathogenesis of several airway diseases such as allergic rhinitis and asthma (Barnes et al., Am. Rev. Respir. Dis., 144:1187-1198 (1991); Barnes et al., Am. Rev. Respir. Dis. 144:1391-1399 (1991); Solway et al., J. Appl. Physiol. 71:2077-2087 (1991); and Joos et al., Eur. Respir. J. 7:1161-1171 (1994)). The most studied lung neuropeptides are the tachykinins substance P and neurokinin A which are released from sensory C-fiber afferents by a variety of stimuli including organic irritants (Nielsen, Crit. Rev. Toxicol. 21:183-208 (1991)), ozone (Hazbun et al., Am. J. Respir. Cell. Mol. Biol. 9:568-572 (1993)), and allergen (Nieber et al., J. Allergy Clin. Immunol. 90:646-652 (1992)). These neuropeptides bind to their receptors, present on a variety of cell types in upper and lower airways, and mediate various effects that contribute to asthmatic airway dysfunction. Such effects include bronchoconstriction (Joos et al., Eur. Respir. J. 7:1161-1171 (1994)), mucus hypersecretion (Rogers et al., Euro. J. Pharmacol. 174:283-286 (1989)), increased vascular permeability (Lundberg et al., Acta Physiol. Scand. 120:217-227 (1984); McDonald et al., J. Neurocytol. 17:605-628 (1988)), chemoattraction and activation of inflammatory cells (Haines et al., J. Immunol. 151:1491-1499 (1993); Numao et al., J. Immunol. 149:3309-3315 (1992); Bost et al., Am. J. Physiol. 262:C537-C545 (1992)), and stimulation of cytokine production (Lotz et al., Science 241:1218-1221 (1988); McGillis et al., Ann. NY Acad. Sci. 594:85-94 (1990)).

Airway hyperresponsiveness is a characteristic pathophysiological feature of bronchial asthma, as well as other respiratory conditions. This altered airway function can be mediated by an allergic airway inflammatory response typically characterized by infiltration of the bronchial wall with eosinophils. Upon activation, these cells release toxic products such as major basic protein which alters muscarinic M2 receptor function resulting in increased release of acetylcholine and increased bronchoconstriction (Fryer et al., 1998, Am. J. Respir. Crit. Care Med. 158:S154-160). Alternatively, non-adrenergic non-cholinergic (NANC) mechanisms have been described as additional neural pathways that control airway smooth muscle tone in asthma (Barnes, 1996, J. Allergy Clin. Immunol. 98:S73-83). Mediators of the NANC nervous system include a variety of sensory neuropeptides that are released from unmyelinated C-fiber afferents by mechanical and chemical stimuli, generating an antidromic stimulation and a local axon reflex which lead to non-cholinergic bronchoconstriction, plasma extravasation, and mucus hypersecretion. These NANC excitatory responses are mediated predominantly by the tachykinins substance P and neurokinin A.

Calcitonin gene-related peptide (CGRP) is another neuropeptide that co-localizes with substance P in some but not all sensory C-fiber afferents in the airways (Lundberg et al., Eur. J. Pharmacol. 108:315-319 (1985); Martling, Acta Physiol. Scand. Suppl. 563:1-57 (1987)). CGRP is a 37 amino acid peptide (the amino acid sequence for human CGRP can be found, for example, under Entrez AccessionNo. 223948; entry 1005250A) that arises from alternative processing of the precursor mRNA encoded by the peptide hormone calcitonin gene (Amara et al., Nature 298:240-244 (1982); Rosenfeld et al., Nature 304:129-135 (1983)). CGRP is a potent vasodilator with long-lasting effects but seems to have little effect on mucus secretion (Brain et al., Nature 313:54-56 (1985); Webber et al., Br. J. Pharmacol. 102:79-84 (1991)). Apparently due to its vasodilator activity, CGRP has been reported to enhance substance P-mediated protein extravasation but it has no direct effects on vascular permeability in the airways (Gamse et al., Eur. J. Pharmacol. 114:61-66 (1985); Lundberg et al., Eur. J. Pharmacol. 108:315-319 (1985)).

Unlike substance P, CGRP is also found in pulmonary neuroepithelial bodies, consisting of innervated clusters of neuroepithelial cells localized within the bronchial epithelium mostly at the branching points of intrapulmonary airways (Cadieux et al., Neuroscience 19:605-627 (1986); Scheuerman, Int. Rev. Cytol. 106:35-88 (1987)). Beneath the epithelium, CGRP-immunoreactive nerve fibers can be demonstrated in the airway submucosa to be in close contact with the epithelium and smooth muscles (Uddman et al., Cell Tissue Res. 241:551-555 (1985); Verastegui et al., Eur. J. Histochem. 41:119-126 (1997)). This unique distribution may suggest a role for this neuropeptide as an important modulator of airway function during exposure to environmental irritants and allergens. However, the exact role of CGRP in the pathophysiological processes that characterize allergic airway hyperresponsiveness remains largely unknown.

CGRP has been frequently reviewed as a mediator of the excitatory NANC nervous system (Barnes, 1991, Am. Rev. Respir. Dis. 143(3 Pt 2): S28-32), because it was described in earlier studies as a potent bronchoconstrictor of human airways *in vitro* (Palmer et al., Thorax 40:713 (1985); Palmer et al., Br. J. Pharmacol. 91:95-101 (1987)). However, no bronchoconstrictor effects could be demonstrated for CGRP in other studies using guinea pig or human airways (Kröll et al, 1990, J. Appl. Physiol. 68(4): 1679-1687; Lundberg et al., Eur. J. Pharmacol. 108:315-319 (1985); Martling et al., Regul. Pept. 20:125-139 (1988)). Moreover, CGRP is known to activate adenylate cyclase and to increase cAMP, an event usually associated with bronchodilation, and unlike tachykinins, CGRP does not induce mucus hypersecretion or plasma extravasation.

However, several additional studies have continued to support a conclusion that CGRP mediates bronchoconstriction and inflammation in the airways. For example, Forsythe et al. concluded that mast cells from patients with chronic cough have an increased responsiveness to CGRP (significantly more histamine release was induced in lavage cells from cough and cough variant asthma) (Forsythe et al., 2000, Clin. Exp. Allergy 30:225-232). Nagase et al. concluded that CGRP enhanced dry gas hyperpnea challenge (HC)-induced bronchoconstriction in guinea pigs (Nagase et al., 1996, Am. J. Respir. Crit. Care Med. 154:1551-1556). Kanazawa et al. concluded that CGRP antagonized the protective effect of adrenomedullin on histamine-induced bronchoconstriction in guinea pigs (Kanazawa et al., 1996, Clin. Exp. Pharmacol. Physiol. 23:472-475). Bellibas concluded that CGRP was capable of causing eosinophilia in the lung of rats and that it may contribute to airway inflammation in the lungs of patients with asthma (Bellibas, 1996, Peptides 17(3):563-564). Zhu et al. concluded that although other neuropeptides could induce relaxation of equine smooth muscle, CGRP did not induce relaxation (Zhu et al., 1997, Am. J. Physiol. 273:L997-1001). A recent study has demonstrated that exogenous CGRP inhibits substance P-induced bronchoconstriction of normal guinea pig airways and carbamylcholine-induced bronchoconstriction of isolated normal human airways (Cadieux et al., Am. J. Respir. Crit. Care Med. 159:235-243 (1999)). However, CGRP was found in the same study to be *ineffective* against the constriction induced by these agonists in ovalbumin-sensitized guinea pig airways and human peripheral airways showing some evidence of inflammatory cellular infiltrates, thus causing the authors to conclude that the ability of CGRP to "limit the extent of airwayhyperresponsiveness is strongly impaired in inflammatory conditions." PCT Publication No. WO 97/09046 describes the use of CGRP receptor antagonists to inhibit treat or prevent diseases mediated by CGRP, among which asthma is listed.

CGRP has been proposed as useful for the treatment of certain conditions. For example, U.S. Patent No. 5,910,482 to Yillampalli et al. proposes the use of CGRP to treat or prevent preeclampsia or eclampsia. U.S. Patent No. 5,958,877 to Wimalawansa proposes the use of CGRP to treat vasospasms, ischemia, renal failure and male impotence. Both of these patents are based on reports that CGRP induces vasodilation.

Two patents to Vignery, U.S. Patent No. 5,858,978 and 5,635,478, describe the use of CGRP to inhibit the release of the cytokines, IL-1, or IL-1 and IL-2, from immune cells, and specifically, from macrophages and lymphocytes. These patents broadly suggest that CGRP can be used to treat a wide variety of conditions involving inflammation by inhibiting the proinflammatory release of IL-1, or IL-1 and IL-2. Vigneryteaches that such conditions include pain, orthopedic dysfunction, viral diseases, edema, arthritis, diseases of the urinary tract and of joints, autoimmune diseases, anaphylactic conditions, shock and allergic reactions, with asthma being mentioned among the allergic reactions. However, with regard to allergic inflammation, the suggestion to inhibit the release of IL-1 or IL-1 and IL-2 in patient with allergic inflammation such as allergic asthma, is not consistent with, and in fact is contrary to, what is known about allergic inflammation by those of skill in the art. More specifically, it is known in the art that the allergic inflammation that is characteristic of conditions such as allergic asthma is mediated by a T helper type 2 (Th2) response, which involves the release of cytokines (primarily by mast cells and eosinophils) such as IL-4, IL-5 and IL-13, and which can generally be downregulated by the opposing cytokines of a T helper type 1 (Th1) immune response, such as IFNγ and IL-12 (*e.g.*, Cohn et al., 1998, J. Immunol. 161: 3813-3816; Hofstra et al., 1998, J. Immunol. 161:5054-5060; Cohn et al., 2000, Pharmacol. Ther. 88:187-196; Wong et al., 2000, Biochem. Pharmacol.59:1323-1335; Mazzarella et al., 2000, Allergy 55(61):6-9). IL-12 is produced by macrophages (in addition to IL-1 and IL-6) and IFNγ, a stimulator of macrophage activity, is produced by Th1-type lymphocytes (in addition to IL-2). Therefore, the inhibition of these immune cells and cytokines would not be expected by those of skill in the art to be useful to treat allergic inflammation, in contrast to the teachings of Vignery. Indeed, there have been studies that demonstrate that production of IL-12 and IL-10 by alveolar macrophages is valuable in the resolution of allergic inflammation (*e.g.,* Magnan et al., 1998, Allergy 53:1092-1095), and that the stimulation of lung macrophages suppresses allergic inflammation (Tang etal., 2001, J. Immunol. 166:1471-1481). U.S. Patent No. 5,674,483 to Tu et al. demonstrates that IL-12 inhibits inflammation and airwayhyperresponsiveness. Finally, the present inventors have demonstrated experimentally that the inhibition of IL-1 *in vivo* does not have any effect on airway hyperresponsiveness, via anti-IL-1 administration, IL-1 receptor antagonists or IL-1 knockout mice (data not shown). Therefore, one of skill in the art of allergic inflammation and particularly, allergic inflammation of the respiratory system, would not, based on the teachings of Vignery, look to the use of CGRP to treat allergic inflammation, and in fact would be dissuaded from doing so.

Therefore, prior to the present invention, the role of CGRP in airway hyperresponsiveness was inconclusive, and at best, CGRP was *not* thought to be an effective or desirable candidate for use for treatment of airway constriction during inflammatory conditions.

### SUMMARY OF THE INVENTION

One embodiment of the present invention relates to compositions for use in inhibiting airway hyperresponsiveness in a mammal, including the step of administering to a mammal an agent that binds to and activates a calcitonin gene related peptide (CGRP) receptor in the lungs of the mammal, wherein the mammal has, or is at risk of developing, airway hyperresponsiveness. The airway hyperresponsiveness is allergen-induced airway hyperresponsiveness. In this embodiment, the mammal has been sensitized to an allergen and has been exposed to, or is at risk of being exposed to, an amount of the allergen that is sufficient to induce airway hyperresponsiveness (AHR) in the mammal in the absence of the agent. Also disclosed herein is a step of monitoring the mammal to detect whether AHR in the mammal is inhibited, wherein if AHR is detected in the mammal, additional amounts of the agent are administered until AHR is not detected in the mammal. Preferably, the mammal is a human.

In one embodiment, the agent is to be administered within a time period of between 48 hours or less prior to exposure to an AHR provoking stimulus that is sufficient to induce AHR, and within 48 hours or less after the detection of the first symptoms of AHR. In one aspect, the agent is administered upon the detection of the first symptoms of AHR. In another aspect, the agent is administered within 1 hour after the detection of the first symptoms of AHR. In another aspect, the agent is administered within 12 hours or less prior to exposure to a AHR provoking stimulus that is sufficient to induce AHR. In another aspect, the agent is administered within 2 hours or less prior to exposure to a AHR provoking stimulus that is sufficient to induce AHR. In another aspect, the agent is to be administered to the mammal every one to two days.

The agent includes CGRP, a fragment of CGRP that binds to and activates a CGRP receptor, and a homologue of CGRP that binds to and activates a CGRP receptor. In another aspect, the agent is an antibody that selectively binds to and activates the CGRP receptor. The antibody can include a divalent antibody, or a bivalent antibody, wherein the antibody selectively binds to the CGRP receptor and to an antigen on a cell selected from the group consisting of a lung smooth muscle cell and a lung epithelial cell. In another aspect, the agent is an antigen binding fragment of an antibody that selectively binds to an activates the CGRP receptor.

In one embodiment, the agent is administered at a dose of from 0.1µg x kilogram⁻¹ and 20 µg x kilogram⁻¹ body weight of the mammal. In another embodiment, the agent is administered at a dose of from 0.1 µg x kilogram⁻¹ and 10 µg x kilogram⁻¹ body weight of the mammal. In another embodiment, the agent is administered at a dose of from 0.1µg x kilogram⁻¹ and 5 µg x kilogram⁻¹ body weight of the mammal.

In one embodiment, the agent is targeted to cells in the lung of the mammal selected from the group consisting of smooth muscle cells and epithelial cells. Preferably, the agent is administered by direct delivery of the agent to the lung of the mammal, such as by aerosol delivery although the agent can be delivered by parenteral delivery. In one aspect, the agent is administered by oral delivery.

Preferably, the administration of the agent reduces the airway hyperresponsiveness of the mammal such that the FEY₁ value of the mammal is improved by at least about 5%. More preferably, the administration of the agent prevents airway hyperresponsiveness in the mammal when administered prior to exposure of the mammal to a AHR provoking stimulus that is sufficient to induce AHR.

In one embodiment, the agent is administered to the mammal in conjunction with another agent selected from the group consisting of: corticosteroids (oral, inhaled and injected), β-agonists (long or short acting), leukotriene modifiers (inhibitors or receptor antagonists), antihistamines, phosphodiesterase inhibitors, sodium cromoglycate, nedocrimal, and theophylline. In another embodiment, the agent is administered to the mammal in conjunction with a CGRP receptor activity modifying protein (RAMP). In another embodiment, the agent is administered in a pharmaceutically acceptable excipient.

Another embodiment of the present invention relates to a method to identify an agent for reducing airway hyperresponsiveness in a mammal according to claim 26. The method includes the steps of: (a) contacting a calcitonin gene related peptide (CGRP) receptor with a putative regulatory agent; (b) detecting whether the putative regulatory agent binds to the CGRP receptor; (c) administering a putative regulatory agent which binds to the CGRP receptor to a non-human test mammal in which airway hyperresponsiveness can be induced and detecting whether the putative regulatory agent reduces airway hyperresponsiveness in the test mammal upon induction of airway hyperresponsiveness in the presence of the putative regulatory agent as compared to in the absence of the putative regulatory agent. Putative regulatory agents that bind to the CGRP receptor and that reduce airway hyperresponsiveness in the test mammal are identified as agents which reduce airway hyperresponsiveness. In one embodiment, step (c) of administering comprises administering the putative regulatory agent which binds to the CGRP receptor to a non-human test mammal that has been sensitized to an allergen and detecting whether the putative regulatory agent reduces airway hyperresponsiveness in the test mammal when the mammal is challenged with the allergen, as compared to in the absence of the putative regulatory agent. Putative regulatory agents that bind to the CGRP receptor and that reduce airway hyperresponsiveness in the test mammal are identified as agents which reduce allergen-induced airway hyperresponsiveness.

In one aspect of this method, the CGRP receptor is a soluble receptor. In another aspect, in part (a), the CGRP receptor is expressed by a cell, and wherein the step (b) of detecting further comprises detecting whether the CGRP receptor is activated by the putative regulatory compound. In another aspect, the non-human test mammal is a mouse. The putative regulatory agent can be a product of rational drug design. In another aspect, the putative regulatory agent is an antibody.

### BRIEF DESCRIPTION OF THE DRAWINGS OF THE INVENTION

Fig. 1A is a bar graph showing the effect of treatment of sensitized mice with anti-VLA-4 and anti-IL5 antibodies prior to allergen challenge on the numbers of total cells (TOT), macrophages (MAC), eosinophils (EOS), neutrophils (NEU) and lymphocytes (LYM) in the bronchoalveolar lavage (BAL) fluid.
Figs. 1B and 1C are line graphs showing the effect on lung resistance (Fig. 1B) and dynamic compliance (Fig 1C) by treatment of sensitized mice with anti-VLA-4 and anti-IL5 antibodies prior to allergen challenge.
Figs 2A and 2B are line graphs showing the CGRP immunoreactivity in central intrapulmonary airways as compared to the numbers of BAL eosinophils (Fig. 2A) and tissue infiltrating eosinophils (Fig. 2B).
Figs. 3A and 3B are line graphs showing the effects of treatment of mice with CGRP(8-37) and exogenous CGRP at 2h prior to each allergen challenge on lung resistance (Fig. 3A) and dynamic compliance (Fig. 3B).
Figs. 3C and 3D are bar graphs showing the effects of treatment of mice with CGRP(8-37) and exogenous CGRP at 2h prior to each allergen challenge on numbers of cells in BAL (Fig. 3C) and tissue infiltrating eosinophils (Fig. 3D).
Figs. 4A and 4B are line graphs showing the effect of intraperitoneal administration of exogenous CGRP (α-CGRP) after the period of allergen challenge and at 2 h prior to the assessment of airway function, on lung resistance (Fig. 4A) and dynamic compliance (Fig. 4B).
Figs. 5A and 5B are line graphs showing the effect of exposure to aerosolized CGRP (10⁻⁶ M) after the period of allergen challenge and at 2 h prior to the assessment of airway function, on lung resistance (Fig. 5A) and dynamic compliance (Fig. 5B).

### DETAILED DESCRIPTION OF THE INVENTION

The present inventors have used a mouse model of ovalbumin-induced airway inflammation to investigate the role of calcitonin gene-related peptide (CGRP) in the development of airway hyperresponsiveness (AHR), and have demonstrated that activation of the CGRP receptor in the lungs of an animal is a powerful means of reducing airway hyperresponsiveness in the animal, and is particularly useful for the treatment of allergen-induced airway hyperresponsiveness. The results herein show that CGRP is normally expressed in the bronchial epithelium and submucosal nerve plexuses of central and peripheral intrapulmonary airways in normal mice. Allergen challenge induced a characteristic eosinophilic airway inflammation and caused a significant depletion of CGRP in the airways of sensitized mice with subsequent development of increased airway responsiveness to methacholine. Treatment with anti-VLA4 or anti-IL5, which blocked the recruitment of eosinophils, prevented allergen-mediated depletion of CGRP and abolished the subsequent development of AHR in sensitized and challenged mice. Administration of a CGRP receptor antagonist (peptide fragment 8-37) prior to each allergen challenge or after the challenge period did not alter the development of AHR in these animals. However, administration of exogenous CGRP prior to each allergen challenge to compensate for the *in vivo* depletion, resulted in a complete suppression of AHR. Similarly, administration of CGRP after the allergen challenge period also completely abolished AHR, an effect that was neutralized by a prior treatment with CGRP receptor antagonist. These data demonstrate that allergen exposure can mediate a significant depletion of CGRP in an eosinophil-dependent manner that results in the subsequent development of AHR in sensitized animals. Thus, the present inventors believe that bronchial epithelium-derived CGRP plays a critical role in modulating the development of allergic AHR.

As demonstrated in the Examples section, a CGRP receptor antagonist had no effect on airway responsiveness to methacholine, thus clearly demonstrating that endogenous CGRP does not contribute to the development of AHR in sensitized mice. In contrast, administration of exogenous CGRP into sensitized mice prior to allergen challenge or after, just before the assessment of airway function, fully restored normal airway responsiveness to inhaled methacholine, an effect that was neutralized by a pretreatment of mice with the receptor antagonist CGRP(8-37). Thus, CGRP can act through its putative receptor(s), *downstream* of the allergic inflammatory cascade that leads to the development of AHR, to restore normal airway tone. As such, the present invention is useful for the treatment of AHR associated with conditions other than allergic inflammation.

In contrast with prior studies which have concluded either that CGRP is a constrictor or that CGRP is ineffective at reducing constriction of airways under inflammatory conditions, the present inventors have demonstrated that CGRP, given either by intraperitoneal or by an inhalation route, completely abolished AHR to methacholine in mice that were sensitized and challenged with ovalbumin, and which developed a characteristic allergic airway eosinophilic inflammation. The present inventors have further demonstrated that this effect was mediated by CGRP receptor(s) since it was neutralized by pretreatment of mice with the specific receptor antagonist CGRP(8-37). The present inventors have demonstrated that sensitization and allergen challenge of the airways does not alter CGRP receptor function but rather the production of CGRP itself.

To demonstrate the effects of CGRP on airway hyperresponsiveness, the present inventors have used an established mouse model of AHR, as previously described, for example, in Takeda et al., (1997). J. Exp. Med. 186, 449-454. This non-human model system is an antigen-driven murine system that is characterized by an immune (IgE) response, a dependence on a Th2-type response, and an eosinophil response, and that mimics human allergic inflammation of the airways. The model is characterized by both a marked and evolving hyperresponsiveness of the airways. The use of this mouse to investigate airway hyperresponsiveness is described in detail in the Examples section.

One embodiment of the present invention relates to compositions for use in a method to inhibit airway hyperresponsiveness in a mammal, comprising administering to a mammal an agent that binds to and activates a calcitonin gene related peptide (CGRP) receptor in the lungs of the mammal, wherein the mammal has, or is at risk of developing, airway hyperresponsiveness. The airway hyperresponsiveness is allergen-induced airway hyperresponsiveness. Preferably, the mammal has been sensitized to the allergen and has been exposed to, or is at risk of being exposed to, an amount of the allergen that is sufficient to induce airway hyperresponsiveness in the absence of the agent.

According to the present invention, "airway hyperresponsiveness" or "AHR" refers to an abnormality of the airways that allows them to narrow too easily and/or too much in response to a stimulus capable of inducing airflow limitation. AHR can be a functional alteration of the respiratory system resulting from inflammation in the airways or airway remodeling (*e.g.*, such as by collagen deposition). Airflow limitation refers to narrowing of airways that can be irreversible or reversible. Airflow limitation or airway hyperresponsiveness can be caused by collagen deposition, bronchospasm, airway smooth muscle hypertrophy, airway smooth muscle contraction, mucous secretion, cellular deposits, epithelial destruction, alteration to epithelial permeability, alterations to smooth muscle function or sensitivity, abnormalities of the lung parenchyma and infiltrative diseases in and around the airways. Many of these causative factors can be associated with inflammation. AHR can be triggered in a patient with a condition associated with the above causative factors by exposure to a provoking agent or stimulus, also referred to herein as an AHR provoking stimulus. Such stimuli include an allergen, methacholine, a histamine, a leukotriene, saline, hyperventilation, exercise, sulfur dioxide, adenosine, propranolol, cold air, an antigen, bradykinin, acetylcholine, a prostaglandin, ozone, environmental air pollutants and mixtures thereof. The present invention is directed to allergen-induced airway hyperresponsiveness associated with any respiratory condition.

AHR can be measured by a stress test that comprises measuring a mammal's respiratory system function in response to a provoking agent (*i.e.,* stimulus). AHR can be measured as a change in respiratory function from baseline plotted against the dose of a provoking agent (a procedure for such measurement and a mammal model useful therefore are described in detail below in the Examples). Respiratory function can be measured by, for example, spirometry, plethysmograph, peak flows, symptom scores, physical signs (*i.e.,* respiratory rate), wheezing, exercise tolerance, use of rescue medication *(i.e.,* bronchodilators), cough and blood gases. In humans, spirometry can be used to gauge the change in respiratory function in conjunction with a provoking agent, such as methacholine or histamine. In humans, spirometry is performed by asking a person to take a deep breath and blow, as long, as hard and as fast as possible into a gauge that measures airflow and volume. The volume of air expired in the first second is known as forced expiratory volume (FEV₁) and the total amount of air expired is known as the forced vital capacity (FVC). In humans, normal predicted FEV₁ and FVC are available and standardized according to weight, height, sex and race. An individual free of disease has an FEV₁ and a FVC of at least about 80% of normal predicted values for a particular person and a ratio of FEV₁/FVC of at least about 80%. Values are determined before (*i.e*, representing a mammal's resting state) and after (*i.e.*, representing a mammal's higher lung resistance state) inhalation of the provoking agent. The position of the resulting curve indicates the sensitivity of the airways to the provoking agent.

The effect of increasing doses or concentrations of the provoking agent on lung function is determined by measuring the forced expired volume in 1 second (FEV₁) and FEV₁ over forced vital capacity (FEV₁/FVC ratio) of the mammal challenged with the provoking agent. In humans, the dose or concentration of a provoking agent *(i.e.,* methacholine or histamine) that causes a 20% fall in FEV₁ (PC₂₀FEV₁) is indicative of the degree of AHR. FEV₁ and FVC values can be measured using methods known to those of skill in the art.

Pulmonary function measurements of airway resistance (R_{L}) and dynamic compliance (C_{L}) and hyperresponsiveness can be determined by measuring transpulmonary pressure as the pressure difference between the airway opening and the body plethysmograph. Volume is the calibrated pressure change in the body plethysmograph and flow is the digital differentiation of the volume signal. Resistance (R_{L}) and compliance (C_{L}) are obtained using methods known to those of skill in the art (*e.g.*, such as by using a recursive least squares solution of the equation of motion). The measurement of lung resistance (R_{L}) and dynamic compliance (C₁) are described in detail in the Examples. It should be noted that measuring the airway resistance (R_{L}) value in a non-human mammal (*e.g.*, a mouse) can be used to diagnose airflow obstruction similar to measuring the FEV₁ and/or FEV₁/FVC ratio in a human.

A variety of provoking agents are useful for measuring AHR values. Suitable provoking agents include direct and indirect stimuli, and are typically provoking agents that trigger AHR *in vivo.* As used herein, the phrase "provoking agent" can be used interchangeably with the phrase "AHR provoking stimulus". Preferred provoking agents or stimulus include, for example, an allergen, methacholine, a histamine, organic irritants, irritating gases and chemicals, a leukotriene, saline, hyperventilation, exercise, sulfur dioxide, adenosine, propranolol, cold air, an antigen, bradykinin, acetylcholine, a prostaglandin, ozone, environmental air pollutants and mixtures thereof. Preferably, for experimental induction of AHR, methacholine (Mch) is used as a provoking agent. Preferred concentrations of Mch to use in a concentration-response curve are between 0.001 and 100 milligram per milliliter (mg/ml). More preferred concentrations of Mch to use in a concentration-response curve are between 0.01 and 50 mg/ml. Even more preferred concentrations of Mch to use in a concentration-response curve are between 0.02 and 25 mg/ml. When Mch is used as a provoking agent, the degree of AHR is defined by the provocative concentration of Mch needed to cause a 20% drop of the FEV₁ of a mammal (PC_{20methacholine}FEV₁). For example, in humans and using standard protocols in the art, a normal person typically has a PC_{20methacholine}FEV₁ >8 mg/ml of Mch. Thus, in humans, AHR is defined as PC_{20methacholine}FEV₁ <8 mg/ml of Mch.

According to the present invention, respiratory function can also be evaluated with a variety of static tests that comprise measuring a mammal's respiratory system function in the absence of a provoking agent. Examples of static tests include, for example, spirometry, plethysmography, peak flows, symptom scores, physical signs (*i.e.,* respiratory rate), wheezing, exercise tolerance, use of rescue medication (*i.e.,* bronchodilators), blood gases and cough. Evaluating pulmonary function in static tests can be performed by measuring, for example, Total Lung Capacity (TLC), Thoracic Gas Volume (TgV), Functional Residual Capacity (FRC), Residual Volume (RV) and Specific Conductance (SGL) for lung volumes, Diffusing Capacity of the Lung for Carbon Monoxide (DLCO), arterial blood gases, including pH, P_{O2} and P_{CO2} for gas exchange. Both FEV₁ and FEV₁/FVC can be used to measure airflow limitation. If spirometry is used in humans, the FEV₁ of an individual can be compared to the FEV₁ of predicted values. Predicted FEV₁ values are available for standard normograms based on the animal's age, sex, weight, height and race. A normal mammal typically has an FEV₁ at least about 80% of the predicted FEV₁ for the mammal. Airflow limitation results in a FEV₁ or FVC of less than 80% of predicted values. An alternative method to measure airflow limitation is based on the ratio of FEV₁ and FVC (FEV₁/FVC). Disease free individuals are defined as having a FEV₁/FVC ratio of at least about 80%. Airflow obstruction causes the ratio of FEV₁/FVC to fall to less than 80% of predicted values. Thus, a mammal having airflow limitation is defined by an FEV₁/FVC less than about 80%.

As used herein, to reduce airway hyperresponsiveness refers to any measurable reduction in airway hyperresponsiveness and/or any reduction of the occurrence or frequency with which airway hyperresponsiveness occurs in a patient. A reduction in AHR can be measured using any of the above-described techniques or any other suitable method known in the art. Preferably, airway hyperresponsiveness, or the potential therefor, is reduced, optimally, to an extent that the mammal no longer suffers discomfort and/or altered function resulting from or associated with airway hyperresponsiveness. To prevent airway hyperresponsiveness refers to preventing or stopping the induction of airway hyperresponsiveness before biological characteristics of airway hyperresponsiveness as discussed above can be substantially detected or measured in a patient.

In one embodiment, the compositions of the present invention decrease methacholine responsiveness in the mammal. Preferably, the compositions of the present invention result in an improvement in a mammal's PC_{20methacholine}FEV₁ value such that the PC_{20methacholine}FEV₁ value obtained before use of the present compositions when the mammal is provoked with a first concentration of methacholine is the same as the PC_{20methacholine}FEV₁ value obtained after use of the present compositions when the mammal is provoked with double the amount of the first concentration of methacholine. Preferably, the compositions of the present invention result in an improvement in a mammal's PC_{20methacholine}FEV₁ value such that the PC_{20methacholine}FEV₁ value obtained before the use of the present compositions when the mammal is provoked with between 0.01 mg/ml to 8 mg/ml of methacholine is the same as the PC_{20methacholine}FEV₁ value obtained after the use of the present compositions when the mammal is provoked with between 0.02 mg/ml to 16 mg/ml of methacholine.

In another embodiment, the compositions of the present invention improve a mammal's FEV₁ by at least 5%, and more preferably by between 6% and 100%, more preferably by between 7% and 100%, and even more preferably by between 8% and 100% of the mammal's predicted FEV₁. In another embodiment, the compositions of the present invention improve a mammal's FEV₁ by at least 5%, and preferably, at least 10%, and even more preferably, at least 25%, and even more preferably, at least 50%, and even more preferably, at least 75%.

In yet another embodiment, the compositions of the present invention result in an increase in the PC_{20methacholine}FEV₁of a mammal by one doubling concentration towards the PC_{20methacholine}FEV₁ of a normal mammal. A normal mammal refers to a mammal known not to suffer from or be susceptible to abnormal AHR. A patient, or test mammal refers to a mammal suspected of suffering from or being susceptible to abnormal AHR.

Therefore, a mammal that has airway hyperresponsiveness is a mammal in which airway hyperresponsiveness can be measured or detected, wherein the airway hyperresponsiveness is typically induced by exposure to an AHR provoking stimulus, as described above. Similarly, a mammal that has allergen-induced airway hyperresponsiveness is a mammal in which airway hyperresponsiveness can be measured or detected, wherein the airway hyperresponsiveness is induced by exposure to an allergen. To be induced by an AHR provoking stimulus, such as an allergen, the airway hyperresponsiveness is apparently or obviously, directly or indirectly triggered by (*e.g.*, caused by, a symptom of, indicative of, concurrent with) an exposure to the stimulus. Symptoms of AHR include indicators of altered respiratory function (described in detail above), change in respiratory rate, wheezing, lowered exercise tolerance, cough and altered blood gases.

In the case of an allergen, the airway hypenesponsiveness is apparently or obviously, directly or indirectly triggered by an allergen to which a mammal has previously been sensitized. Sensitization to an allergen refers to being previously exposed one or more times to an allergen such that an immune response is developed against the allergen. Responses associated with an allergic reaction (*e.g.*, histamine release, rhinitis, edema, vasodilation, bronchial constriction, airway inflammation), typically do not occur when a naive individual is exposed to the allergen for the first time, but once a cellular and humoral immune response is produced against the allergen, the individual is "sensitized" to the allergen. Allergic reactions then occur when the sensitized individual is re-exposed to the same allergen (*e.g.*, an allergen challenge). Once an individual is sensitized to an allergen, the allergic reactions can become worse with each subsequent exposure to the allergen, because each re-exposure not only produces allergic symptoms, but further increases the level of antibody produced against the allergen and the level of T cell response against the allergen.

Typically, conditions associated with allergic responses to antigens (*i.e.,* allergens) are at least partially characterized by inflammation of pulmonary tissues. Such conditions or diseases are discussed above. However, it is noted that the present invention is specifically directed to compositions for use in the treatment of AHR, and not to the treatment of the condition or causative factor that caused the AHR, such as allergic inflammation (i.e., the present method acts downstream of allergic inflammation, for example). Indeed, the use of the present invention is fully effective to reduce AHR even after the inflammatory response in the lungs of the mammal is fully established. A mammal that is at risk of developing airway hyperresponsiveness is a mammal that has been exposed to, or is at risk of being exposed to, an AHR provoking stimulus that is sufficient to trigger AHR, but does not yet display a measurable or detectable characteristic or symptom of airway hyperresponsiveness, such symptoms being described previously herein. A mammal that is at risk of developing allergen-induced airway hyperresponsiveness is a mammal that has been previously sensitized to an allergen, and that has been exposed to, or is at risk of being exposed to, an amount of the allergen that is sufficient to trigger AHR *(i.e.,* a triggering, or challenge dose of allergen), but does not yet display a measurable or detectable characteristic or symptom of airway hyperresponsiveness. A mammal that is at risk of developing airway hyperresponsiveness also includes a mammal that is identified as being predisposed to or susceptible to such a condition or disease.

Inflammation is typically characterized by the release of inflammatory mediators (e.g., cytokines or chemokines) which recruit cells involved in inflammation to a tissue. A condition or disease associated with allergic inflammation is a condition or disease in which the elicitation of one type of immune response (*e.g.*, a Th2-type immune response) against a sensitizing agent, such as an allergen, can result in the release of inflammatory mediators that recruit cells involved in inflammation in a mammal, the presence of which can lead to tissue damage and sometimes death. As discussed previously, a Th2-type immune response is characterized in part by the release of cytokines which include IL-4, IL-5 and IL-13. Airway hyperresponsiveness can occur in a patient that has, or is at risk of developing, any chronic obstructive disease of the airways, including asthma, chronic obstructive pulmonary disease, allergic bronchopulmonary aspergillosis, hypersensitivity pneumonia, eosinophilic pneumonia, emphysema, bronchitis, allergic bronchitis bronchiectasis, cystic fibrosis, tuberculosis, hypersensitivity pneumonitis, occupational asthma, sarcoid, reactive airway disease syndrome, interstitial lung disease, hyper-eosinophilic syndrome, rhinitis, sinusitis, exercise-induced asthma, pollution-induced asthma, cough variant asthma, and parasitic lung disease. The method of the present invention is particularly useful for treating allergen-induced airway hyperresponsiveness, and most particularly, allergen-induced asthma, in addition to other forms of airway hyperresponsiveness.

According to the use of the present invention, an effective amount of an agent that inhibits AHR (also referred to simply as "an agent") to administer to a mammal comprises an amount that is capable of reducing airway hyperresponsiveness (AHR) without being toxic to the mammal. An amount that is toxic to a mammal comprises any amount that causes damage to the structure or function of a mammal (*i.e.,* poisonous).

In one embodiment, the effectiveness of an AHR inhibiting agent to protect a mammal from AHR in a mammal having or at risk of developing AHR can be measured in doubling amounts. For example, the ability of a mammal to be protected from AHR (*i.e.,* experience a reduction in or a prevention of) by administration of a given agent is significant if the mammal's PC_{20methacholine}FEV₁ is at 1 mg/ml before administration of the agent and is at 2 mg/ml of Mch after administration of the agent. Similarly, an agent is considered effective if the mammal's PC_{20methacholine}FEV₁ is at 2 mg/ml before administration of the agent and is at 4 mg/ml of Mch after administration of the agent.

In one embodiment of the present invention, in a mammal that has AHR, an effective amount of an agent to administer to a mammal is an amount that measurably reduces AHR in the mammal as compared to prior to administration of the agent. In another embodiment, an effective amount of an agent to administer to a mammal is an amount that measurably reduces AHR in the mammal as compared to a level of airway AHR in a population of mammals with inflammation that is associated with AHR wherein the agent was not administered. The agent that binds to and activates a CGRP receptor according to the present invention is preferably capable of reducing AHR in a mammal, even when the agent is administered after the onset of the physical symptoms of AHR. Most preferably, an effective amount of the agent is an amount that reduces the symptoms of AHR to the point where AHR is no longer detected in the patient. In another embodiment, an effective amount of AHR is an amount that prevents, or substantially inhibits the onset of AHR when the agent is administered prior to exposure of the patient to an AHR provoking stimulus, such as an allergen, in a manner sufficient to induce AHR in the absence of the agent.

In one embodiment of the present invention, an effective amount of an agent to administer to a mammal includes an amount that is capable of decreasing methacholine responsiveness without being toxic to the mammal. A preferred effective amount of an agent comprises an amount that is capable of increasing the PC_{20methacholine}FEV₁ of a mammal treated with the an agent by one doubling concentration towards the PC_{20methacholine}FEV₁ of a normal mammal. A normal mammal refers to a mammal known not to suffer from or be susceptible to abnormal AHR. A non-human test mammal refers to a mammal suspected of suffering from or being susceptible to abnormal AHR.

In another embodiment, an effective amount of an agent according to the use of the present invention, comprises an amount that results in an improvement in a mammal's PC_{20methacholine}FEV₁ value such that the PC_{20methacholine}FEV₁ value obtained before administration of the an agent when the mammal is provoked with a first concentration of methacholine is the same as the PC_{20methacholine}FEV₁ value obtained after administration of the an agent when the mammal is provoked with double the amount of the first concentration of methacholine. A preferred amount of an agent comprises an amount that results in an improvement in a mammal's PC_{20methacholine}FEV₁ value such that the PC_{20methacholine}FEV₁ value obtained before administration of the an agent is between 0.01 mg/ml to about 8 mg/ml of methacholine is the same as the PC_{20methacholine}FEV₁ value obtained after administration of the an agent is between 0.02 mg/ml to 16 mg/ml of methacholine.

As previously described herein the effectiveness of an agent to protect a mammal having or susceptible to AHR can be determined by measuring the percent improvement in FEV₁ and/or the FEV₁/FVC ratio before and after administration of the agent. In one embodiment, an effective amount of an agent comprises an amount that is capable of reducing the airflow limitation of a mammal such that the FEV₁/FVC value of the mammal is at least 80%. In another embodiment, an effective amount of an agent comprises an amount that is capable of reducing the airflow limitation of a mammal such that the FEV₁/FVC value of the mammal is improved by at least 5%, or at least 100cc or PGFRG 10L/min. In another embodiment, an effective amount of an agent comprises an amount that improves a mammal's FEV₁ by at least 5%, and more preferably by between 6% and 100%, more preferably by between 7% and 100%, and even more preferably by between 8% and 100% (or 200 ml) of the mammal's predicted FEV₁. In another embodiment, an effective amount of an agent comprises an amount that improves a mammal's FEV₁ by at least 5%, and preferably, at least 10%, and even more preferably, at least 25%, and even more preferably, at least 50%, and even more preferably, at least 75%.

A static test can be performed before or after administration of a provocative agent used in a stress test. Static tests have been discussed in detail above.

A suitable single dose of an agent of the present invention to be administered to a mammal is a dose that is capable of reducing or preventing airway hyperresponsiveness in a mammal when administered one or more times over a suitable time period. In particular, a suitable single dose of an agent comprises a dose that improves AHR by a doubling dose of a provoking agent or improves the static respiratory function of a mammal. A preferred single dose of an agent comprises between 0.01 microgram x kilogram⁻¹ and 10 milligram x kilogram⁻¹ body weight of a mammal. A more preferred single dose of an agent comprises between 0.1µg x kilogram⁻¹ and 20 µg x kilogram⁻¹ body weight of said mammal. Another preferred single dose of an agent comprises between 0.1 µg x kilogram⁻¹ and 10 µg x kilogram⁻¹ body weight of said mammal. Another preferred single dose of an agent comprises between 0.1 µg x kilogram⁻¹ and 5 µg x kilogram⁻¹ body weight of said mammal. Another preferred single dose of an agent comprises between 1 microgram x kilogram⁻¹ and 10 milligram x kilogram⁻¹ body weight of a mammal. Another preferred single dose of an agent comprises between 5 microgram x kilogram⁻¹ and 7 milligram x kilogram⁻¹ body weight of a mammal. Another preferred single dose of an agent comprises between 10 microgram x kilogram⁻¹ and 5 milligram x kilogram⁻¹ body weight of a mammal. If the agent is delivered by aerosol or parenterally, a particularly preferred single dose of an agent comprises between 0.01 microgram x kilogram⁻¹ and 10 milligram x kilogram⁻¹ body weight of a mammal, and more preferably between 0.01 milligram x kilogram⁻¹ and 5 milligram x kilogram⁻¹ body weight of a mammal, and preferably between 0.01 milligram x kilogram⁻¹ and 1 milligram x kilogram⁻¹ bodyweight of a mammal, and more preferably between 0.1µg x kilogram⁻¹ and 20 µg x kilogram⁻¹ body weight of said mammal, and more preferably, between 0.1 µg x kilogram⁻¹ and 10 µg x kilogram⁻¹ body weight of said mammal, and more preferably, between 0.1 µg x kilogram⁻¹ and 5 µg x kilogram⁻¹ body weight of said mammal. Typically, the agent can be administered in smaller doses when delivered by aerosol, as compared to other routes of delivery.

One of skill in the art will be able to determine that the number of doses of an agent to be administered to a mammal is dependent upon the extent of the airway hyperresponsiveness and the underlying condition of which AHR is a symptom, and the response of an individual patient to the treatment. In addition, the clinician will be able to determine the appropriate timing for delivery of the agent in a manner effective to reduce AHR in the mammal. Preferably, the agent is delivered within 48 hours prior to exposure of the patient to an amount of an AHR provoking stimulus effective to induce AHR, and more preferably, within 36 hours, and more preferably within 24 hours, and more preferably within 12 hours, and more preferably within 6 hours, 5 hours, 4 hours, 3 hours, 2 hours, or 1 hour of prior to exposure of the patient to an amount of AHR provoking stimulus effective to induce AHR. In one embodiment, the agent is to be administered as soon as it is recognized (*i.e.*, immediately) by the patient or clinician that the patient has been exposed or is to be exposed to an AHR provoking stimulus, and especially an AHR provoking stimulus to which the patient is sensitized (i.e., an allergen). In another embodiment, the agent is administered upon the first sign of development of AHR, and preferably, within at least 2 hours of the development of symptoms of AHR, and more preferably, within at least 1 hour, and more preferably within at least 30 minutes, and more preferably within at least 10 minutes, and more preferably within at least 5 minutes of development of symptoms of AHR. Symptoms of AHR and methods for measuring or detecting such symptoms have been described in detail above. Preferably, such administrations are given once every 1-2 hours until signs of reduction of AHR appear, and then as needed until the symptoms of AHR are gone. In one embodiment, the agent of the present invention can be administered on a regular basis as a prophylactic treatment for the prevention of AHR, or minimally, to reduce the risk of developing AHR. Prophylactic administration protocols can be developed by the clinician and will depend on the dosage and general need and health of the individual patient, but generally, administration every 1-7 days is contemplated as being sufficient to inhibit AHR in the individual.

The compositions of the present invention can be used in any animal, and particularly, in any animal of the Vertebrate class, Mammalia, including primates, rodents, livestock and domestic pets. Preferred mammals to treat using the compositions of the present invention include humans.

In accordance with the present invention, acceptable protocols to administer an agent including the route of administration and the effective amount of an agent to be administered to a mammal can be accomplished by those skilled in the art. An agent of the present invention can be administered *in vivo* or *ex vivo.* Suitable *in vivo* routes of administration can include oral, nasal, inhaled, topical, intratracheal, transdermal, rectal, and parenteral routes. Preferred parenteral routes can include subcutaneous, intradermal, intravenous, intramuscular, and intraperitoneal routes. Preferred topical routes include inhalation by aerosol (*i.e.,* spraying) or topical surface administration to the skin of a mammal. Preferably, an agent is to be administered by nasal, inhaled (*e.g.*, aerosol), intratracheal, oral, topical, or intraperitoneal routes. *Ex vivo* refers to performing part of the administration step outside of the patient, such as by contacting a population of cells removed from a patient with an agent that binds to and activates a CGRP receptor, and then returning the contacted cells to the patient. *Ex vivo* methods are particularly suitable when the cell to which the agent is to be delivered can easily be removed from and returned to the patient. *In vitro* and ex *vivo* routes of administration of a composition to a culture of cells can be accomplished by a method including transfection, transformation, electroporation, microinjection, lipofection, adsorption, protoplast fusion, use of protein carrying agents, use of ion carrying agents, use of detergents for cell permeabilization, and simply mixing (*e.g.*, combining) a compound in culture with a target cell.

Aerosol (inhalation) delivery can be performed using methods standard in the art (see, for example, Stribling et al., Proc. Natl. Acad. Sci. USA 189:11277-11281, 1992). Oral delivery can be performed by complexing a therapeutic composition of the present invention to a carrier capable of withstanding degradation by digestive enzymes in the gut of an animal. Examples of such carriers include plastic capsules or tablets, such as those known in the art. Such routes can include the use of pharmaceutically acceptable carriers as described in more detail below.

According to the present invention, administration of an agent useful in the present method activates a CGRP receptor in the lungs of a mammal. It is desirable to modulate airway hyperresponsiveness in the mammal to obtain a therapeutic benefit in the mammal *(i.e.,* the patient). Patients whom are suitable candidates for the uses of the present invention include any patient who has been sensitized to an allergen, and who is experiencing, or is at risk of experiencing, airway hyperresponsiveness due to an exposure to the allergen. These patients include patients that have any allergen-induced disease or condition of the airways, such as allergen-induced asthma.

Accordingly, the present invention includes the use of a variety of agents *(i.e.,* regulatory compounds) according to claim 1 which, by increasing or replacing the amount of CGRP in the lungs of the mammal, and particularly by acting directly on the CGRP receptor to activate the receptor, increase or restore the biological activity of the CGRP receptor in a cell such that airway hyperresponsiveness is reduced in a mammal. Agents useful in the present invention include, for example, proteins, nucleic-acid molecules antibodies (including antigen-binding fragments), and compounds that are products of rational drug design (*i.e.,* drugs).

Agents for use in the present invention are CGRP receptor agonists according to claim 1. A CGRP receptor agonist is any agent which increases or restores the biological activity of a CGRP receptor *(i. e.,* as compared to the biological activity of the CGRP receptor prior to contact with such agent, preferably by direct binding to and activation of the receptor). Such a compound is effective to agonize the biological activity of a CGRP receptor, for example by binding to and activating the receptor for CGRP. The phrase "CGRP receptor agonist" generally refers to any compound (agent), including an antibody that selectively binds to and activates or increases the activation of a CGRP receptor, CGRP, CGRP homologues, and any suitable product of drug design (*e.g.,* a mimetic of CGRP) which is characterized by its ability to agonize (*e.g.*, stimulate, induce, increase, enhance, activate) the biological activity of a naturally occurring CGRP receptor (*e.g.*, by interaction/binding with and/or activation of a CGRP receptor).

In general, the biological activity or biological action of a protein refers to any function(s) exhibited or performed by the protein that is ascribed to the naturally occurring form of the protein as measured or observed *in vivo* (*i.e.,* in the natural physiological environment of the protein) or *in vitro* (*i.e.,* under laboratory conditions). Modifications of a protein, such as in a homologue or mimetic (discussed below), may result in proteins having the same biological activity as the naturally occurring protein, or in proteins having decreased or increased biological activity as compared to the naturally occurring protein. Modifications which result in a decrease in protein expression or a decrease in the activity of the protein, can be referred to as inactivation (complete or partial), down-regulation, or decreased action of a protein. Similarly, modifications which result in an increase in protein expression or an increase in the activity of the protein, can be referred to as amplification, overproduction, activation, enhancement, up-regulation or increased action of a protein.

As used herein,"CGRP receptor biological activity" refers to a biological activity that can include:(a) binding to CGRP; and (b) responding to contact with CGRP or another suitable stimulator by mediating one or more activities in a cell expressing the receptor, including increasing cAMP, increasing intracellular calcium mobilization and phosphorylation of the receptor. For example, human CGRP receptor biological activity can be identified using bioassays and molecular assays, including calcium mobilization assays, phosphorylation assays, kinase assays, immunofluorescence microscopy, and combinations thereof. Alternatively, a CGRP receptor of the present invention can be identified by its ability to bind CGRP, such as in any standard binding assay (*e.g.*, competitive binding techniques, equilibrium dialysis or BIAcore methods).

Therefore, one agent for use in the present invention is an isolated CGRP peptide that is capable of binding to and activating a CGRP receptor in the lungs of a mammal. As used herein, reference to an isolated protein or peptide, including an isolated CGRP protein, generally includes full-length proteins, fusion proteins, and fragments of such proteins. CGRP occurs in two known forms (α and β) in the human. The α and β-strains of CGRP have been isolated and fully characterized by amino acid sequencing and fast atom bombardment-mass spectrometry (FABMS) (Wimalawansa, S. J., Morris, H. R., Etienne, A., Blench, I., Panico, M., and Maclntyre, I. Isolation, purification and characterization of b -hCGRP from human spinal cord, Biochem. Biophys. Res. Commun., 167,993 (1990); Steenberg, et al. FEBS Letts. 183:403 (1985)). The nucleic acid and amino acid sequence of human CGRP are described in U.S. Patent Nos. 4,736,023 and 4,549,986, respectively. In addition, methods of synthetically producing human CGRP are described in detail therein. In addition, the nucleic acid and amino acid sequences for CGRP from a variety of mammalian species can be found in public databases (see, for example, Entrez Accession Nos: AAA00500 (human CGRP from U.S. Patent No. 4,549,986); XP006209, TCHU and NP001732 (human CGRP α); XP006016, P10092, A25864 (human CGRP β); AAK16431 (mouse CGRP α); AAK06841 (mouse CGRP β); A44173 (rat CGRP β); CAB97487 (dog CGRP); and P31888 (sheep CGRP)). The human synthetic calcitonin gene-related peptide (α-CGRP) used in the experiment of the present invention was obtained from Sigma Chemical Co. (St. Louis, MO). It is noted that the sequence homology of CGRP between mammalian species is very high. For example, the mouse, rat and human CGRP peptides can be used interchangeably among species, as was done in the Examples (i.e., human into mouse).

According to the present invention, a fragment of a CGRP peptide that is useful in the present invention is any fragment that binds to and still activates a CGRP receptor. The native human CGRP peptide is 37 amino acids in length and therefore, one of skill in the art can readily produce and test fragments of CGRP that can serve as CGRP receptor agonists. It is noted that one fragment of CGRP (amino acid positions 8-37) is actually an antagonist of CGRP receptors and thus is not considered to be a CGRP receptor agonist for the present invention.

The present invention also includes a fusion protein that includes a CGRP-containing domain *(i.e.,* an amino acid sequence for a CGRP peptide according to the present invention) attached to one or more fusion segments. Suitable fusion segments for use with the present invention include segments that can: enhance a protein's stability; provide other desirable biological activity; and/or assist with the purification of a CGRP peptide (*e.g.*, by affinity chromatography). A suitable fusion segment can be a domain of any size that has the desired function (*e.g.*, imparts increased stability, solubility, action or biological activity; and/or simplifies purification of a protein). Fusion segments can be joined to amino and/or carboxyl termini of the CGRP-containing domain of the protein and can be susceptible to cleavage in order to enable straight-forward recovery of the CGRP. Fusion proteins are preferably produced by culturing a recombinant cell transfected with a fusion nucleic acid molecule that encodes a protein including the fusion segment attached to either the carboxyl and/or amino terminal end of a CGRP-containing domain.

A CGRP protein of the present invention (including protein homologues or mimetics of CGRP) may be produced by any method suitable for the production of proteins or polypeptides. A particularly preferred method for production of a CGRP protein of the present invention is by chemical synthesis methods. For example, such methods include well known chemical procedures, such as solution or solid-phase peptide synthesis, or semi-synthesis in solution beginning with protein fragments coupled through conventional solution methods. Such methods are well known in the art and may be found in general texts and articles in the area such as: Merrifield, 1997, Methods Enzymol. 289:3-13; Wade et al., 1993, Australas Biotechnol. 3(6):332-336; Wong et al., 1991, Experientia 47(11-12):1123-1129; Carey et al., 1991, Ciba Found Symp. 158:187-203; Plaue et al., 1990, Biologicals 18(3):147-157; Bodanszky, 1985, Int. J. Pept. Protein Res. 25(5):449-474; or H. Dugas and C. Penney, BIOORGANIC CHEMISTRY, (1981) at pages 54-92. For example, peptides may be synthesized by solid-phase methodology utilizing a commercially available peptide synthesizer and synthesis cycles supplied by the manufacturer. One skilled in the art recognizes that the solid phase synthesis could also be accomplished using the FMOC strategy and a TFA/scavenger cleavage mixture.

If larger quantities of a CGRP protein are desired, the protein can be produced using recombinant DNA technology, although for proteins of this smaller size (*i.e.,* peptides), peptide synthesis may be generally preferred. A protein can be produced recombinantly by culturing a cell capable of expressing the protein (*i.e.,* by expressing a recombinant nucleic acid molecule encoding the protein, described in detail below) under conditions effective to produce the protein, and recovering the protein. Effective culture conditions include effective media, bioreactor, temperature, pH and oxygen conditions that permit protein production. An effective medium refers to any medium in which a cell is cultured to produce a CGRP protein of the present invention. Such medium typically comprises an aqueous medium having assimilable carbon, nitrogen and phosphate sources, and appropriate salts, minerals, metals and other nutrients, such as vitamins. Recombinant cells *(i.e.,* cells expressing a nucleic acid molecule encoding a CGRP protein) can be cultured in conventional fermentation bioreactors, shake flasks, test tubes, microtiter dishes, and petri plates. Culturing can be carried out at a temperature, pH and oxygen content appropriate for a recombinant cell. Such culturing conditions are within the expertise of one of ordinary skill in the art. Such techniques are well known in the art and are described, for example, in Sambrook et al., 1988, Molecular Cloning: A Laboratory Manual, Cold Spring Harbor Press, Cold Spring Harbor Laboratory, Cold Spring Harbor, New York or Current Protocols in Molecular Biology (1989) and supplements. As discussed elsewhere herein, the nucleic acid and amino acid sequence of CGRP for several mammals have been elucidated and are in the public domain.

Indeed, in one embodiment, the CGRP protein or other protein homologue that activates the CGRP receptor can be provided as a nucleic acid molecule encoding the protein. According to the present invention, a nucleic acid molecule can include DNA, RNA, or derivatives of either DNA or RNA. A nucleic acid molecule of the present invention can include a ribozyme which specifically targets RNA encoding a CGRP receptor. A nucleic acid molecule encoding a CGRP protein or homologue thereof (including protein mimetics) can be obtained from its natural source, either as an entire (i.e., complete) gene or a portion thereof that is capable of encoding a CGRP protein or homologue thereof that increases the activity of a CGRP receptor and thereby reduces AHR, when such protein and/or nucleic acid molecule encoding such protein is administered to the mammal. In one embodiment of the present invention, a nucleic acid molecule encoding CGRP is an oligonucleotide that encodes a portion of CGRP. Such an oligonucleotide can include all or a portion of a regulatory sequence of a nucleic acid molecule encoding CGRP. A nucleic acid molecule can also be produced using recombinant DNA technology (e.g., polymerase chain reaction (PCR) amplification, cloning) or chemical synthesis. Nucleic acid molecules include natural nucleic acid molecules and homologues thereof natural allelic variants and modified nucleic acid molecules in which nucleotides have been inserted, deleted, substituted, and/or inverted in such a manner that such modifications do not substantially interfere with the nucleic acid molecule's ability to encode CGRP or a homologue thereof that is useful in the present invention. An isolated, or biologically pure, nucleic acid molecule, is a nucleic acid molecule that has been removed from its natural milieu. As such, "isolated" and "biologically pure" do not necessarily reflect the extent to which the nucleic acid molecule has been purified.

A nucleic acid molecule homologue can be produced using a number of methods known to those skilled in the art (see, for example, Sambrook et al., Molecular Cloning: A Laboratory Manual, Cold Spring Harbor Labs Press, 1989). For example, nucleic acid molecules can be modified using a variety of techniques including classic mutagenesis techniques and recombinant DNA techniques, such as site-directed mutagenesis, chemical treatment of a nucleic acid molecule to induce mutations, restriction enzyme cleavage of a nucleic acid fragment, ligation of nucleic acid fragments, polymerase chain reaction (PCR) amplification and/or mutagenesis of selected regions of a nucleic acid sequence, synthesis of oligonucleotide mixtures and ligation of mixture groups to "build" a mixture of nucleic acid molecules and combinations thereof. Nucleic acid molecule homologues can be selected from a mixture of modified nucleic acids by screening for the function of the protein encoded by the nucleic acid (e.g., CGRP activity, as appropriate). Techniques to screen for CGRP activity are known to those of skill in the art and have been described elsewhere herein.

Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding CGRP or a homologue thereof. In addition, the phrase "recombinant molecule" primarily refers to a nucleic acid molecule operatively linked to a transcription control sequence, but can be used interchangeably with the phrase "nucleic acid molecule" which is administered to a mammal.

As described above, a nucleic acid molecule encoding CGRP or a homologue thereof that is useful in the present invention can be operatively linked to one or more transcription control sequences to form a recombinant molecule. The phrase "operatively linked" refers to linking a nucleic acid molecule to a transcription control sequence in a manner such that the molecule is able to be expressed when transfected (i.e., transformed, transduced or transfected) into a host cell. Transcription control sequences are sequences which control the initiation, elongation, and termination of transcription. Particularly important transcription control sequences are those which control transcription initiation, such as promoter, enhancer, operator and repressor sequences. Suitable transcription control sequences include any transcription control sequence that can function in a recombinant cell useful for the expression of CGRP or a homologue thereof, and/or useful to administer to a mammal in the present invention. A variety of such transcription control sequences are known to those skilled in the art. Preferred transcription control sequences include those which function in mammalian, bacterial, or insect cells, and preferably in mammalian cells. More preferred transcription control sequences include simian virus 40 (SV-40), β-actin, retroviral long terminal repeat (LTR), Rous sarcoma virus (RSV), cytomegalovirus (CMV), *tac, lac, trp, trc,* oxy-pro, omp/lpp, rrnB, bacteriophage lambda (λ) (such as λp_{L} and λp_{R} and fusions that include such promoters), bacteriophage T7, *T7lac,* bacteriophage T3, bacteriophage SP6, bacteriophage SP01, metallothionein, alpha mating factor, *Pichia* alcohol oxidase, alphavirus subgenomic promoters (such as Sindbis virus subgenomic promoters), baculovirus, *Heliothis zea* insect virus, vaccinia virus and other poxviruses, herpesvirus, and adenovirus transcription control sequences, as well as other sequences capable of controlling gene expression in eukaryotic cells. Additional suitable transcription control sequences include tissue-specific promoters and enhancers (e.g., T cell-specific enhancers and promoters). Transcription control sequences of the present invention can also include naturally occurring transcription control sequences naturally associated with a gene encoding CGRP useful in a method of the present invention.

Recombinant molecules of the present invention, which can be either DNA or RNA, can also contain additional regulatory sequences, such as translation regulatory sequences, origins of replication, and other regulatory sequences that are compatible with the recombinant cell. In one embodiment, a recombinant molecule of the present invention also contains secretory signals (i.e., signal segment nucleic acid sequences) to enable an expressed CGRP or a homologue thereof to be secreted from a cell that produces the protein. Preferred signal segments include signal segments naturally associated with any of the heretofore mentioned CGRP or a homologue thereof.

One or more recombinant molecules of the present invention can be used to produce an encoded product (i.e., CGRP or a homologue thereof). In one embodiment, an encoded product is produced by expressing a nucleic acid molecule of the present invention under conditions effective to produce the protein. A preferred method to produce an encoded protein is by transfecting a host cell with one or more recombinant molecules having a nucleic acid sequence encoding CGRP or a homologue thereof to form a recombinant cell. Suitable host cells to transfect include any cell that can be transfected. Host cells can be either untransfected cells or cells that are already transformed with at least one nucleic acid molecule. Host cells useful in the present invention can be any cell capable of producing CGRP or a homologue thereof, including bacterial, fungal, mammal, and insect cells. A preferred host cell includes a mammalian cell.

According to the present invention, a host cell can be transfected *in vivo* (i.e., by delivery of the nucleic acid molecule into a mammal), *ex vivo* (i.e., outside of a mammal for reintroduction into the mammal, such as by introducing a nucleic acid molecule into a cell which has been removed from a mammal in tissue culture, followed by reintroduction of the cell into the mammal); or *in vitro* (i.e., outside of a mammal, such as in tissue culture for production of a recombinant CGRP or a homologue thereof). Transfection of a nucleic acid molecule into a host cell can be accomplished by any method by which a nucleic acid molecule can be inserted into the cell. Transfection techniques include transfection, electroporation, microinjection, lipofection, adsorption, and protoplast fusion. Preferred methods to transfect host cells *in vivo* include lipofection, viral vector delivery and adsorption.

A recombinant cell of the present invention comprises a host cell transfected with a nucleic acid molecule that encodes CGRP or a homologue thereof. It may be appreciated by one skilled in the art that use of recombinant DNA technologies can improve expression of transfected nucleic acid molecules by manipulating, for example, the number of copies of the nucleic acid molecules within a host cell, the efficiency with which those nucleic acid molecules are transcribed, the efficiency with which the resultant transcripts are translated, and the efficiency of post-translational modifications. Recombinant techniques useful for increasing the expression of nucleic acid molecules encoding CGRP or a homologue thereof include operatively linking nucleic acid molecules to high-copy number plasmids, integration of the nucleic acid molecules into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Dalgarno sequences), modification of nucleic acid molecules to correspond to the codon usage of the host cell, and deletion of sequences that destabilize transcripts. The activity of an expressed recombinant CGRP or a homologue thereof may be improved by fragmenting, modifying, or derivatizing nucleic acid molecules encoding such a protein.

Another agent for use in the present invention includes CGRP analogs which are agonists of CGRP receptor activity. Such analogs are defined herein as homologues or mimetics of a naturally occurring CGRP protein, wherein such compound (the analog) has substantially the same or increased biological activity as compared to the naturally occurring CGRP peptide (*i.e*., prototype) upon which the homologue or mimetic is based. Such an agonist is typically sufficiently similar in structure to CGRP that it is capable of such biological activity. As used herein, the term "homologue" is used to refer to a peptide which differs from a naturally occurring peptide (*i*.*e.,* the "prototype") by minor modifications to the naturally occurring peptide, but which maintains the basic peptide and side chain structure of the naturally occurring form. Such changes include changes in one or a few amino acid side chains; changes in one or a few amino acids, including deletions (*e.g*., a truncated version of the peptide) insertions and/or substitutions; changes in stereochemistry of one or a few atoms; and/or minor derivatizations, including methylation, glycosylation, phosphorylation, acetylation, myristoylation, prenylation, palmitation, amidation and/or addition of glycosylphosphatidyl inositol. Preferably, a homologue that is an agonist has substantially the same or enhanced biological activity as compared to the naturally occurring protein.

Suitable homologues of the present invention can be identified in a straightforward manner by the ability of the homologue to bind to a CGRP receptor, such as in any standard binding assay. A CGRP homologue can also be identified by its ability to activate the CGRP receptor, which can be measured experimentally by any of the methods described previously herein, including measurement of cAMP activity, calcium mobilization, and phosphorylation of the receptor. Another method to evaluate a CGRP homologue for utility in the present method is to confirm the ability of the compound to reduce AHR in a test animal as described previously herein. Various agonist homologues of CGRP are known in the art and are described, for example, in U.S. Patent No. 4,697,002 to Kempe (substitutions at position 36); U.S. Patent No. 4,687,839 to Kempe (D-amino acid substitutions at minimally positions 36 and 37); and U.S. Patent No. 4,530,838 to Evans et al. (substitutions at position 35). Methods for determining the biological activity of CGRP are described in these patents and can be used to evaluate other CGRP homologues.

A mimetic refers to any peptide or non-peptide compound that is able to mimic the biological action of a naturally occurring peptide, often because the mimetic has a basic structure that mimics the basic structure of the naturally occurring peptide and/or has the salient biological properties of the naturally occurring peptide. Mimetics can include peptides that have substantial modifications from the prototype such as no side chain similarity with the naturally occurring peptide (such modifications, for example, may decrease its susceptibility to degradation); anti-idiotypic and/or catalytic antibodies, or fragments thereof; non-proteinaceous portions of an isolated protein (*e.g*., carbohydrate structures); or synthetic or natural organic molecules, including nucleic acids and drugs identified through combinatorial chemistry, for example. Such mimetics can be designed, selected and/or otherwise identified using a variety of methods known in the art. Various methods of drug design, useful to design mimetics or other therapeutic compounds useful in the present invention are disclosed in Maulik et al., 1997, *supra*, and are discussed below in detail.

CGRP receptor agonists referred to herein include, for example, compounds that are products of rational drug design, natural products, and compounds having partially defined CGRP properties. A CGRP receptor agonist can be a protein-based compound, a carbohydrate-based compound, a lipid-based compound, a nucleic acid-based compound, a natural organic compound, a synthetically derived organic compound, an antibody, or antigen binding fragments thereof. In one embodiment, CGRP agonists of the present invention include drugs, including peptides, oligonucleotides, carbohydrates and/or synthetic organic molecules which bind to and regulate activity (*e.g*., activate) the CGRP receptor. Such an agent can be obtained, for example, from molecular diversity strategies (a combination of related strategies allowing the rapid construction of large, chemically diverse molecule libraries), libraries of natural or synthetic compounds, in particular from chemical or combinatorial libraries (*i.e*., libraries of compounds that differ in sequence or size but that have the same building blocks) or by rational drug design. See for example, Maulik et al., 1997, Molecular Biotechnology: Therapeutic Application and Strategies, Wiley-Liss, Inc.

In a molecular diversity strategy, large compound libraries are synthesized, for example, from peptides, oligonucleotides, carbohydrates and/or synthetic organic molecules, using biological, enzymatic and/or chemical approaches. The critical parameters in developing a molecular diversity strategy include subunit diversity, molecular size, and library diversity. The general goal of screening such libraries is to utilize sequential application of combinatorial selection to obtain high-affinity ligands against a desired target, and then optimize the lead molecules by either random or directed design strategies. Methods of molecular diversity are described in detail in Maulik, et al., *supra.*

In a rational drug design procedure, the three-dimensional structure of a regulatory compound can be analyzed by, for example, nuclear magnetic resonance (NMR) or X-ray crystallography. This three-dimensional structure can then be used to predict structures of potential compounds, such as potential regulatory agents by, for example, computer modeling. The predicted compound structure can be used to optimize lead compounds derived, for example, by molecular diversity methods. In addition, the predicted compound structure can be produced by, for example, chemical synthesis, recombinant DNA technology, or by isolating a mimetope from a natural source (*e.g*., plants, animals, bacteria and fungi).

Various other methods of structure-based drug design are disclosed in Maulik et al., 1997, *supra.* Maulik et al. disclose, for example, methods of directed design, in which the user directs the process of creating novel molecules from a fragment library of appropriately selected fragments; random design, in which the user uses a genetic or other algorithm to randomly mutate fragments and their combinations while simultaneously applying a selection criterion to evaluate the fitness of candidate ligands; and a grid-based approach in which the user calculates the interaction energy between three dimensional receptor structures and small fragment probes, followed by linking together of favorable probe sites.

One agent useful in the method of the present invention includes an antibody or antigen binding fragment that selectively binds to a CGRP receptor. Such an antibody can selectively bind to any CGRP receptor, including fragments of such receptors. According to the present invention, the phrase "selectively binds to" refers to the ability of an antibody, antigen binding fragment or binding partner of the present invention to preferentially bind to specified proteins (*e.g*., a CGRP receptor). More specifically, the phrase "selectively binds" refers to the specific binding of one protein to another (*e.g*., an antibody, fragment thereof, or binding partner to an antigen), wherein the level of binding, as measured by any standard assay (*e.g*., an immunoassay), is statistically significantly higher than the background control for the assay. For example, when performing an immunoassay, controls typically include a reaction well/tube that contain antibody or antigen binding fragment alone (*i.e*., in the absence of antigen), wherein an amount of reactivity (*e.g*., non-specific binding to the well) by the antibody or antigen binding fragment thereof in the absence of the antigen is considered to be background. Binding can be measured using a variety of methods standard in the art including enzyme immunoassays (*e.g*., ELISA), immunoblot assays, etc.

Antibodies are characterized in that they comprise immunoglobulin domains and as such, they are members of the immunoglobulin superfamily of proteins. Generally speaking, an antibody molecule comprises two types of chains. One type of chain is referred to as the heavy or H chain and the other is referred to as the light or L chain. The two chains are present in an equimolar ratio, with each antibody molecule typically having two H chains and two L chains. The two H chains are linked together by disulfide bonds and each H chain is linked to a L chain by a disulfide bond. There are only two types of L chains referred to as lambda (λ) and kappa (κ) chains. In contrast, there are five major H chain classes referred to as isotypes. The five classes include immunoglobulin M (IgM or µ), immunoglobulin D (IgD or δ), immunoglobulin G (IgG or λ), immunoglobulin A (IgA or α), and immunoglobulin E (IgE or ∈). The distinctive characteristics between such isotypes are defined by the constant domain of the immunoglobulin and are discussed in detail below. Human immunoglobulin molecules comprise nine isotypes, IgM, IgD, IgE, four subclasses of IgG including IgG1 (γ1), IgG2 (γ2), IgG3 (γ3) and IgG4 (γ4), and two subclasses of IgA including IgA1 (α1) and IgA2 (α2).

Each H or L chain of an immunoglobulin molecule comprises two regions referred to as L chain variable domains (V_{L} domains) and L chain constant domains (C_{L} domains), and H chain variable domains (V_{H} domains) and H chain constant domains (C_{H} domains). A complete C_{H} domain comprises three sub-domains (CH1, CH2, CH3) and a hinge region. Together, one H chain and one L chain can form an arm of an immunoglobulin molecule having an immunoglobulin variable region. A complete immunoglobulin molecule comprises two associated (*e.g*., di-sulfide linked) arms. Thus, each arm of a whole immunoglobulin comprises a V_{H+L} region, and a C_{H+L} region. As used herein, the term "variable region" or "V region" refers to a V_{H+L} region (also known as an Fv fragment), a V_{L} region or a V_{H} region. Also as used herein, the term "constant region" or "C region" refers to a C_{H+L} region, a C_{L} region or a C_{H} region.

Limited digestion of an immunoglobulin with a protease may produce two fragments. An antigen binding fragment is referred to as an Fab, an Fab', or an F(ab')₂ fragment. A fragment lacking the ability to bind to antigen is referred to as an Fc fragment. An Fab fragment comprises one arm of an immunoglobulin molecule containing a L chain (V_{L} + C_{L} domains) paired with the V_{H} region and a portion of the C_{H} region (CH1 domain). An Fab' fragment corresponds to an Fab fragment with part of the hinge region attached to the CH1 domain. An F(ab')₂ fragment corresponds to two Fab' fragments that are normally covalently linked to each other through a di-sulfide bond, typically in the hinge regions.

The C_{H} domain defines the isotype of an immunoglobulin and confers different functional characteristics depending upon the isotype. For example, µ constant regions enable the formation of pentameric aggregates of IgM molecules and α constant regions enable the formation of dimers.

The antigen specificity of an immunoglobulin molecule is conferred by the amino acid sequence of a variable, or V, region. As such, V regions of different immunoglobulin molecules can vary significantly depending upon their antigen specificity. Certain portions of a V region are more conserved than others and are referred to as framework regions (FW regions). In contrast, certain portions of a V region are highly variable and are designated hypervariable regions. When the V_{L} and V_{H} domains pair in an immunoglobulin molecule, the hypervariable regions from each domain associate and create hypervariable loops that form the antigen binding sites. Thus, the hypervariable loops determine the specificity of an immunoglobulin and are termed complementarity-determining regions (CDRs) because their surfaces are complementary to antigens.

Further variability of V regions is conferred by combinatorial variability of gene segments that encode an immunoglobulin V region. Immunoglobulin genes comprise multiple germline gene segments which somatically rearrange to form a rearranged immunoglobulin gene that encodes an immunoglobulin molecule. V_{L} regions are encoded by a L chain V gene segment and J gene segment (joining segment). V_{H} regions are encoded by a H chain V gene segment, D gene segment (diversity segment) and J gene segment (joining segment).

Both a L chain and H chain V gene segment contain three regions of substantial amino acid sequence variability. Such regions are referred to as L chain CDR1, CDR2 and CDR3, and H chain CDR1, CDR2 and CDR3, respectively. The length of an L chain CDR1 can vary substantially between different V_{L} regions. For example, the length of CDR1 can vary from about 7 amino acids to about 17 amino acids. In contrast, the lengths of L chain CDR2 and CDR3 typically do not vary between different V_{L} regions. The length of a H chain CDR3 can vary substantially between different V_{H} regions. For example, the length of CDR3 can vary from about 1 amino acid to about 20 amino acids. Each H and L chain CDR region is flanked by FW regions.

Other functional aspects of an immunoglobulin molecule include the valency of an immunoglobulin molecule, the affinity of an immunoglobulin molecule, and the avidity of an immunoglobulin molecule. As used herein, affinity refers to the strength with which an immunoglobulin molecule binds to an antigen at a single site on an immunoglobulin molecule (*i.e*., a monovalent Fab fragment binding to a monovalent antigen). Affinity differs from avidity which refers to the sum total of the strength with which an immunoglobulin binds to an antigen. Immunoglobulin binding affinity can be measured using techniques standard in the art, such as competitive binding techniques, equilibrium dialysis or BIAcore methods. As used herein, valency refers to the number of different antigen binding sites per immunoglobulin molecule (*i.e*., the number of antigen binding sites per antibody molecule of antigen binding fragment). For example, a monovalent immunoglobulin molecule can only bind to one antigen at one time, whereas a bivalent immunoglobulin molecule can bind to two or more antigens at one time, and so forth. Both monovalent and bivalent antibodies that selectively bind to CGRP receptors are encompassed herein.

In one embodiment of the present invention, a monovalent antibody can be used as a regulatory compound (discussed below). Such an antibody is not capable of aggregating receptors. Divalent antibodies can also be used in the present invention.

In one embodiment, the antibody is abi- or multi-specific antibody. A bi-specific (or multi-specific) antibody is capable of binding two (or more) antigens, as with a divalent (or multivalent) antibody, but in this case, the antigens are different antigens (*i.e*., the antibody exhibits dual or greater specificity). A bi-specific antibody suitable for use in the present method includes an antibody having: (a) a first portion (*e.g*., a first antigen binding portion) which binds to the CGRP receptor; and (b) a second portion which binds to a cell surface molecule expressed by a cell which expresses a CGRP receptor (*e.g*., a smooth muscle cell, an epithelial cell, or other suitable cell in the lung of the patient). In this embodiment, the second portion can bind to any cell surface molecule. In a preferred embodiment, the second portion is capable of targeting the regulatory antibody to a specific target cell (*i.e*., the regulatory antibody binds to a target molecule). For example, the second portion of the bi-specific antibody can be an antibody that binds to another cell surface molecule on a target cell, such as an epithelial cell. In another embodiment, the bivalent antibody can have a first portion that binds to either the CGRP receptor or the CGRP (or other stimulator) to be delivered, and a second portion that binds to a RAMP that complexes with the CGRP receptor (discussed in detail below).

Isolated antibodies of the present invention can include serum containing such antibodies, or antibodies that have been purified to varying degrees. Whole antibodies of the present invention can be polyclonal or monoclonal. Alternatively, functional equivalents of whole antibodies, such as antigen binding fragments in which one or more antibody domains are truncated or absent (*e.g*., Fv, Fab, Fab', or F(ab)₂ fragments), as well as genetically-engineered antibodies or antigen binding fragments thereof, including single chain antibodies or antibodies that can bind to more than one epitope (*e.g*., bi-specific antibodies), or antibodies that can bind to one or more different antigens (*e.g*., bi- or multi-specific antibodies), may also be employed in the invention.

Genetically engineered antibodies of the invention include those produced by standard recombinant DNA techniques involving the manipulation and re-expression of DNA encoding antibody variable and/or constant regions. Particular examples include, chimeric antibodies, where the V_{H} and/or V_{L} domains of the antibody come from a different source to the remainder of the antibody, and CDR grafted antibodies (and antigen binding fragments thereof), in which at least one CDR sequence and optionally at least one variable region framework amino acid is (are) derived from one source and the remaining portions of the variable and the constant regions (as appropriate) are derived from a different source. Construction of chimeric and CDR-grafted antibodies are described, for example, in European Patent Applications: EP-A 0194276, EP-A 0239400, EP-A 0451216 and EP-A 0460617.

Generally, in the production of an antibody, a suitable experimental animal, such as, for example a rabbit, a sheep, a hamster, a guinea pig, a mouse, a rat, or a chicken, is exposed to an antigen against which an antibody is desired. Typically, an animal is immunized with an effective amount of antigen that is injected into the animal. An effective amount of antigen refers to an amount needed to induce antibody production by the animal. The animal's immune system is then allowed to respond over a pre-determined period of time. The immunization process can be repeated until the immune system is found to be producing antibodies to the antigen. In order to obtain polyclonal antibodies specific for the antigen, serum is collected from the animal that contains the desired antibodies (or in the case of a chicken, antibody can be collected from the eggs). Such serum is useful as a reagent. Polyclonal antibodies can be further purified from the serum (or eggs) by, for example, treating the serum with ammonium sulfate.

Monoclonal antibodies maybe produced according to the methodology ofKohler and Milstein (Nature 256:495-497, 1975). For example, B lymphocytes are recovered from the spleen (or any suitable tissue) of an immunized animal and then fused with myeloma cells to obtain a population of hybridoma cells capable of continual growth in suitable culture medium. Hybridomas producing the desired antibody are selected by testing the ability of the antibody produced by the hybridoma to bind to the desired antigen.

A preferred method to produce antibodies of the present invention includes (a) administering to an animal an effective amount of a protein, peptide or mimetic thereof of the present invention to produce the antibodies and (b) recovering the antibodies. In another method, antibodies of the present invention are produced recombinantly. For example, once a cell line, for example a hybridoma, expressing an antibody according to the invention has been obtained, it is possible to clone therefrom the cDNA and to identify the variable region genes encoding the desired antibody, including the sequences encoding the CDRs. From here, antibodies and antigen binding fragments according to the invention may be obtained by preparing one or more replicable expression vectors containing at least the DNA sequence encoding the variable domain of the antibody heavy or light chain and optionally other DNA sequences encoding remaining portions of the heavy and/or light chains as desired, and transforming/transfecting an appropriate host cell, in which production of the antibody will occur. Suitable expression hosts include bacteria, (for example, an *E. coli* strain), fungi, (in particular yeasts, *e.g*. members of the genera *Pichia, Saccharomyces,* or *Kluyveromyces*,) and mammalian cell lines, *e.g.* a non-producing myeloma cell line, such as a mouse NSO line, or CHO cells. In order to obtain efficient transcription and translation, the DNA sequence in each vector should include appropriate regulatory sequences, particularly a promoter and leader sequence operably linked to the variable domain sequence. Particular methods for producing antibodies in this way are generally well known and routinely used. For example, basic molecular biology procedures are described by Maniatis et al. (Molecular Cloning, Cold Spring Harbor Laboratory, New York, 1989); DNA sequencing can be performed as described in Sanger et al. (PNAS 74, 5463, (1977)) and the Amersham International plc sequencing handbook; and site directed mutagenesis can be carried out according to the method of Kramer et al. (Nucl. Acids Res. 12, 9441, (1984)) and the Anglian Biotechnology Ltd. handbook. Additionally, there are numerous publications, including patent specifications, detailing techniques suitable for the preparation of antibodies by manipulation of DNA, creation of expression vectors and transformation of appropriate cells, for example as reviewed by Mountain A and Adair, J R in Biotechnology and Genetic Engineering Reviews (ed. Tombs, M P, 10, Chapter 1, 1992, Intercept, Andover, UK) and in the aforementioned European Patent Applications.

Alternative methods, employing, for example, phage display technology (see for example US 5969108, US 5565332, US 5871907, US 5858657) or the selected lymphocyte antibody method of US 5627052 may also be used for the production of antibodies and/or antigen fragments of the invention, as will be readily apparent to the skilled individual.

The invention also extends to non-antibody polypeptides, sometimes referred to as binding partners, that have been designed to bind specifically to, and either activate or inhibit as appropriate, a CGRP receptor according to the present invention. Examples of the design of such polypeptides, which possess a prescribed ligand specificity are given in Beste et al. (Proc. Natl. Acad. Sci. 96:1898-1903, 1999).

CGRP receptors are known in the art, and can be produced by recombinant or synthetic methods. The amino acid and nucleic acid sequences of a CGRP receptor are described, for example, in public databases (see, Entrez Accession No. Q16602, Q63118, or AAC41994). The present inventors contemplate the use of any agent that binds to and activates a CGRP receptor, including agents that may bind to the receptor intracellularly. In addition, the present inventors contemplate other methods of increasing CGRP receptor biological activity which may not include the contact of the receptor with an agent that directly binds to and activates the receptor. For example, CGRP receptor activity may be increased in a cell by increasing the expression of the receptor, by increasing the sensitivity of the receptor.

In one embodiment, the agent is formulated in a composition that can additionally include a receptor activity modifying protein (RAMP) which associates with the main component of the CGRP receptor and CGRP peptide, and enhances the activity of the CGRP/receptor complex. In another embodiment, the agent is a bivalent antibody or other binding agent that binds to CGRP or the CGRP receptor, and to a CGRP RAMP. Such an antibody would cross-link and stabilize the association of the RAMP with the complex, thereby increasing the activity of the CGRP receptor. CGRP RAMP are known in the art (see, for example, NP 005847, NP 005845, NP 005846). Briefly, the CGRP receptor occurs essentially as the main CGRP receptor component, which is also referred to as calcitonin receptor-like receptor (CRLR). In order to bind CGRP, the receptor is modified by RAMP which allows CGRP to bind with high affinity and thereby allow the induction of receptor activity (e.g., increase cAMP, calcium mobilization and phosphorylation of the receptor).

In another embodiment, the agent that binds to and activates a CGRP receptor according to the present invention can be administered in conjunction with another compound or agent that is useful for treating allergen-induced airway hyperresponsiveness in the patient. Such an agent, includes: corticosteroids, (oral, inhaled and injected), β-agonists (long or short acting), leukotriene modifiers (inhibitors or receptor antagonists), antihistamines, phosphodiesterase inhibitors, sodium cromoglycate, nedocrimal, and theophylline.

Typically, an agent useful in the present method is formulated into a therapeutic composition. A composition, and particularly a therapeutic composition, of the present invention generally includes a carrier, and preferably, a pharmaceutically acceptable carrier. According to the present invention, a "pharmaceutically acceptable carrier" includes pharmaceutically acceptable excipients and/or pharmaceutically acceptable delivery vehicles, which are suitable for use in administration of the composition to a suitable *in vitro*, *ex vivo* or *in vivo* site. A suitable *in vitro*, in *vivo* or *ex vivo* site is preferably a cell that expresses a CGRP receptor of the present invention, including a smooth muscle cell and an epithelial cell in the lung of the patient. Preferred pharmaceutically acceptable carriers are capable of maintaining a protein, compound, or nucleic acid molecule according to the present invention in a form that, upon arrival of the protein, compound, or nucleic acid molecule at the cell target in a culture or in patient, the protein, compound or nucleic acid molecule is capable of interacting with its target (*e.g*., a naturally occurring CGRP receptor).

Suitable excipients of the present invention include excipients or formularies that transport or help transport, but do not specifically target a composition to a cell (also referred to herein as non-targeting carriers). Examples of pharmaceutically acceptable excipients include water, phosphate buffered saline, Ringer's solution, dextrose solution, serum-containing solutions, Hank's solution, other aqueous physiologically balanced solutions, oils, esters and glycols. Aqueous carriers can contain suitable auxiliary substances required to approximate the physiological conditions of the recipient, for example, by enhancing chemical stability and isotonicity.

Suitable auxiliary substances include, for example, sodium acetate, sodium chloride, sodium lactate, potassium chloride, calcium chloride, and other substances used to produce phosphate buffer, Tris buffer, and bicarbonate buffer. Auxiliary substances can also include preservatives, such as thimerosal, m- or o-cresol, formalin and benzol alcohol. Compositions of the present invention can be sterilized by conventional methods and/or lyophilized.

One type of pharmaceutically acceptable carrier includes a controlled release formulation that is capable of slowly releasing a composition of the present invention into a patient or culture. As used herein, a controlled release formulation comprises a compound of the present invention (*e.g*., a protein (including homologues), a drug, an antibody, a nucleic acid molecule, or a mimetic) in a controlled release vehicle. Suitable controlled release vehicles include biocompatible polymers, other polymeric matrices, capsules, microcapsules, microparticles, bolus preparations, osmotic pumps, diffusion devices, liposomes, lipospheres, and transdermal delivery systems. Other carriers of the present invention include liquids that, upon administration to a patient, form a solid or a gel *in situ.* Preferred carriers are also biodegradable (*i.e*., bioerodible). Natural lipid-containing delivery vehicles include cells and cellular membranes. Artificial lipid-containing delivery vehicles include liposomes and micelles. A delivery vehicle of the present invention can be modified to target to a particular site in a patient, thereby targeting and making use of a compound of the present invention at that site. Suitable modifications include manipulating the chemical formula of the lipid portion of the delivery vehicle and/or introducing into the vehicle a targeting agent capable of specifically targeting a delivery vehicle to a preferred site, for example, a preferred cell type. Other suitable delivery vehicles include gold particles, poly-L-lysine/DNA-molecular conjugates, and artificial chromosomes.

Other suitable carriers include any carrier that can be bound to or incorporated with the agent that extends that half-life of the agent to be delivered. Such a carrier can include any suitable protein carrier or even a fusion segment that extends the half-life of a CGRP peptide, homologue, mimetic, or antibody, for example, when delivered *in vivo.*

A pharmaceutically acceptable carrier which is capable of targeting is herein referred to as a "delivery vehicle." Delivery vehicles of the present invention are capable of delivering a formulation, including a CGRP-receptor activating agent to a target site in a mammal. A "target site" refers to a site in a mammal to which one desires to deliver a therapeutic formulation. For example, a target site can be any cell which is targeted by direct injection or delivery using liposomes or antibodies, such as bi-valent antibodies. A delivery vehicle of the present invention can be modified to target to a particular site in a mammal, thereby targeting and making use of the agent complexed with the liposome at that site. Suitable modifications include manipulating the chemical formula of the lipid portion of the delivery vehicle and/or introducing into the vehicle a compound capable of specifically targeting a delivery vehicle to a preferred site, for example, a preferred cell type. Specifically, targeting refers to causing a delivery vehicle to bind to a particular cell by the interaction of the compound in the vehicle to a molecule on the surface of the cell. Suitable targeting compounds include ligands capable of selectively (*i.e*., specifically) binding another molecule at a particular site. Examples of such ligands include antibodies, antigens, receptors and receptor ligands. Manipulating the chemical formula of the lipid portion of the delivery vehicle can modulate the extracellular or intracellular targeting of the delivery vehicle. For example, a chemical can be added to the lipid formula of a liposome that alters the charge of the lipid bilayer of the liposome so that the liposome fuses with particular cells having particular charge characteristics.

The agent used in the present method can be in any form suitable for delivery, including a liquid, an aerosol, a capsule, a tablet, a pill, a powder, a gel and a granule. Preparations of agents that are particularly suitable for parenteral administration include sterile aqueous or nonaqueous solutions, suspensions or emulsions. Examples of nonaqueous solvents or vehicles are propylene glycol, polyethylene glycol, vegetable oils such as olive oil and injectable organic esters such as ethyl oleate.

In solid dosage forms, the agent can be admixed with at least one inert diluent such as sucrose, lactose or starch. Such dosage forms can also comprise, as is normal practice, additional substances other than inert diluent. In the case of capsules, tablets, and pills, the dosage forms may also comprise buffering agents, pH-sensitive polymers, or any other slow-releasing encapsulants (*i.e*., controlled release vehicles) which are typically used as encapsulating compositions in the food and drug industry or any other controlled release formulations. Tablets and pills can additionally be prepared with an enteric coating.

Liquid dosage forms of agent for oral administration include pharmaceutically acceptable emulsions, solutions, suspensions, syrups and elixirs, containing inert diluents commonly used in the pharmaceutical art. Besides inert diluents, compositions can also include wetting agents, emulsifying, and suspending, and sweetening agents.

Isolated nucleic acid molecules to be administered in the present invention include: (a) isolated nucleic acid molecules useful in the method of the present invention in a non-targeting carrier (e.g., as "naked" DNA molecules, such as is taught, for example in Wolff et al., 1990, Science 247, 1465-1468); and (b) isolated nucleic acid molecules of the present invention complexed to a delivery vehicle of the present invention. Particularly suitable delivery vehicles for local administration of nucleic acid molecules comprise liposomes, viral vectors and ribozymes. Delivery vehicles for local administration can further comprise ligands for targeting the vehicle to a particular site.

One preferred delivery vehicle of the present invention is a liposome. A liposome is capable of remaining stable in an animal for a sufficient amount of time to deliver a nucleic acid molecule described in the present invention to a preferred site in the animal. A liposome, according to the present invention, comprises a lipid composition that is capable of delivering a nucleic acid molecule described in the present invention to a particular, or selected, site in a mammal. A liposome according to the present invention comprises a lipid composition that is capable of fusing with the plasma membrane of the targeted cell to deliver a nucleic acid molecule into a cell. Suitable liposomes for use with the present invention include any liposome. Preferred liposomes of the present invention include those liposomes typically used in, for example, gene delivery methods known to those of skill in the art. More preferred liposomes comprise liposomes having a polycationic lipid composition and/or liposomes having a cholesterol backbone conjugated to polyethylene glycol.

A liposome comprises a lipid composition that is capable of fusing with the plasma membrane of the targeted cell to deliver a nucleic acid molecule or other agent (e.g., a peptide) into a cell. Preferably, the transfection efficiency of a liposome is at least 0.5 microgram (µg) of DNA per 16 nanomole (nmol) of liposome delivered to 10⁶ cells, more preferably at least 1.0 µg of DNA per 16 nmol of liposome delivered to 10⁶ cells, and even more preferably at least 2.0 µg of DNA per 16 nmol of liposome delivered to 10⁶ cells. A preferred liposome is between about 100 and 500 nanometers (nm), more preferably between 150 and 450 nm and even more preferably between 200 and 400 nm in diameter.

Complexing a liposome with a nucleic acid molecule or other agent of the present invention can be achieved using methods standard in the art. A suitable concentration of a nucleic acid molecule or other agent to add to a liposome includes a concentration effective for delivering a sufficient amount of nucleic acid molecule and/or other agent to a cell such that the biological activity of the CGRP receptor is increased in a desired manner. Preferably, nucleic acid molecules are combined with liposomes at a ratio of from 0.1 µg to 10 µg of nucleic acid molecule of the present invention per about 8 nmol liposomes, more preferably from 0.5 µg to 5 µg of nucleic acid molecule per 8 nmol liposomes, and even more preferably 1.0 µg of nucleic acid molecule per 8 nmol liposomes.

Another preferred delivery vehicle comprises a viral vector. A viral vector includes an isolated nucleic acid molecule useful in the method of the present invention, in which the nucleic acid molecules are packaged in a viral coat that allows entrance of DNA into a cell. A number of viral vectors can be used, including those based on alphaviruses, poxviruses, adenoviruses, herpesviruses, lentiviruses, adeno-associated viruses and retroviruses.

Also included in the present invention are therapeutic molecules known as ribozymes. A ribozyme typically contains stretches of complementary RNA bases that can base-pair with a target RNA ligand, including the RNA molecule itself, giving rise to an active site of defined structure that can cleave the bound RNA molecule (See Maulik et al., 1997, *supra*). Therefore, a ribozyme can serve as a targeting delivery vehicle for the nucleic acid molecule.

Another embodiment of the present invention relates to a method to identify an agent for reducing airway hyperresponsiveness in a mammal. The method includes the steps of: (a) contacting a calcitonin gene related peptide (CGRP) receptor with a putative regulatory agent; (b) detecting whether the putative regulatory agent binds to the CGRP receptor; and (c) administering a putative regulatory agent which binds to the CGRP receptor to a non-human test mammal in which AHR can be induced, and detecting whether the putative regulatory agent reduces airway hyperresponsiveness in the test mammal in the presence of the agent as compared to in the absence of the putative regulatory agent. Putative regulatory agents that bind to the CGRP receptor and that reduce airway hyperresponsiveness in the test mammal are identified as agents which reduce airway hyperresponsiveness. In a preferred embodiment, step (c) of administering comprises administering the putative regulatory agent which binds to the CGRP receptor to a non-human test mammal that has been sensitized to an allergen and detecting whether the putative regulatory agent reduces airway hyperresponsiveness in the test mammal when the mammal is challenged with the allergen, as compared to in the absence of the putative regulatory agent. Putative regulatory agents that bind to the CGRP receptor and that reduce airway hyperresponsiveness in the test mammal are identified as agents which reduce allergen-induced airway hyperresponsiveness.

As used herein, the term "putative" refers to compounds having an unknown or previously unappreciated regulatory activity in a particular process. As such, the term "identify" is intended to include all compounds, the usefulness of which as a regulatory compound of CGRP receptor activation for the purposes of reducing airway hyperresponsiveness is determined by a method of the present invention.

In the method of identifying an agent for reducing AHR according to the present invention, the method can be a cell-based assay, or non-cell-based assay. In one embodiment, the CGRP receptor is expressed by a cell (*i.e*., a cell-based assay). In another embodiment the CGRP receptor is in a cell lysate, or is purified or produced free of cells (*e.g*., a soluble CGRP receptor). In accordance with the present invention, a cell-based assay is conducted under conditions which are effective to screen for regulatory compounds useful in the method of the present invention. Effective conditions include appropriate media, temperature, pH and oxygen conditions that permit cell growth. An appropriate, or effective, medium refers to any medium in which a cell of the present invention, when cultured, is capable of cell growth and expression of a CGRP receptor. Such a medium is typically a solid or liquid medium comprising growth factors and assimilable carbon, nitrogen and phosphate sources, as well as appropriate salts, minerals, metals and other nutrients, such as vitamins. Culturing is carried out at a temperature, pH and oxygen content appropriate for the cell. Such culturing conditions are within the expertise of one of ordinary skill in the art.

In one embodiment, the conditions under which a receptor according to the present invention is contacted with a putative regulatory compound, such as by mixing, are conditions in which the receptor is not stimulated (activated) if essentially no regulatory compound is present. For example, such conditions include normal culture conditions in the absence of a stimulatory compound (a stimulatory compound being, *e.g*., the natural ligand for the receptor (CGRP), a stimulatory antibody, or other equivalent stimulus). In this embodiment, the putative regulatory compound is then contacted with the receptor. In this embodiment, the step of detecting is designed to indicate whether the putative regulatory compound binds to the CGRP receptor, and further, whether the putative regulatory compound stimulates the receptor.

The present methods involve contacting cells with the compound being tested for a sufficient time to allow for interaction, activation or inhibition of the receptor by the compound. The cells can naturally express the CGRP receptor, or can recombinantly express a CGRP receptor functional unit. The period of contact with the compound being tested can be varied depending on the result being measured, and can be determined by one of skill in the art. For example, for binding assays, a shorter time of contact with the compound being tested is typically suitable, than when activation is assessed. As used herein, the term "contact period" refers to the time period during which cells are in contact with the compound being tested. The term "incubation period" refers to the entire time during which cells are allowed to grow prior to evaluation, and can be inclusive of the contact period. Thus, the incubation period includes all of the contact period and may include a further time period during which the compound being tested is not present but during which growth is continuing (in the case of a cell based assay) prior to scoring. The incubation time for growth of cells can vary but is sufficient to allow for the binding of the CGRP receptor, activation of the receptor, and/or inhibition of the receptor. It will be recognized that shorter incubation times are preferable because compounds can be more rapidly screened. A preferred incubation time is between about 1 minute to about 48 hours.

The assay of the present invention can also be a non-cell based assay. In this embodiment, the putative regulatory compound can be directly contacted with an isolated receptor, or a receptor component (*e.g*., an isolated extracellular portion of the receptor, or soluble receptor), and the ability of the putative regulatory compound to bind to the receptor or receptor component can be evaluated, such as by an immunoassay or other binding assay. The assay can then include the step of further analyzing whether putative regulatory compounds which bind to a portion of the receptor are capable of increasing the activity of the CGRP receptor. Such further steps can be performed by cell-based assay, as described above, or by non-cell-based assay. For example, isolated membranes may be used to identify compounds that interact with the CGRP receptor being tested. Membranes can be harvested from cells expressing CGRP receptors by standard techniques and used in an *in vitro* binding assay. In one embodiment, a cell that has been transfected with an expresses a CGRP receptor functional unit can be used in whole, or by harvesting the membranes, for screening potential compounds. ¹²⁵I-labeled (other labels can be used also) ligand (*e.g*., ¹²⁵I-labeled CGRP) is contacted with the membranes and assayed for specific activity; specific binding is determined by comparison with binding assays performed in the presence of excess unlabeled ligand. Membranes are typically incubated with labeled ligand in the presence or absence of test compound. Compounds that bind to the receptor and compete with labeled ligand for binding to the membranes reduce the signal compared to the vehicle control samples.

Alternatively, soluble CGRP receptors may be recombinantly expressed and utilized in non-cell based assays to identify compounds that bind to CGRP receptors. Recombinantly expressed CGRP receptor polypeptides or fusion proteins containing an extracellular domain of CGRP receptor can be used in the non-cell based screening assays. Alternatively, peptides corresponding to the extracellular domain of the CGRP receptor or fusion proteins containing the extracellular domain of the CGRP receptor can be used in non-cell based assay systems to identify compounds that bind to the extracellular portion of the CGRP receptor. In non-cell based assays the recombinantly expressed CGRP receptor is attached to a solid substrate such as a test tube, microtiter well or a column, by means well known to those in the art. For example, a CGRP receptor and/or cell lysates containing such receptors can be immobilized on a substrate such as: artificial membranes, organic supports, biopolymer supports and inorganic supports. The protein can be immobilized on the solid support by a variety of methods including adsorption, cross-linking (including covalent bonding), and entrapment. Adsorption can be through van del Waal's forces, hydrogen bonding, ionic bonding, or hydrophobic binding. Exemplary solid supports for adsorption immobilization include polymeric adsorbents and ion-exchange resins. Solid supports can be in any suitable form, including in a bead form, plate form, or well form. The test compounds are then assayed for their ability to bind to the CGRP receptor.

*In vitro* cell based assays may be designed to screen for compounds that regulate CGRP receptor expression at either the transcriptional or translational level. In one embodiment, DNA encoding a reporter molecule can be linked to a regulatory element of the CGRP receptor gene and used in appropriate intact cells, cell extracts or lysates to identify compounds that modulate CGRP receptor gene expression. Appropriate cells or cell extracts are prepared from any cell type that normally expresses the CGRP receptor gene, thereby ensuring that the cell extracts contain the transcription factors required for *in vitro* or *in vivo* transcription. The screen can be used to identify compounds that modulate the expression of the reporter construct. In such screens, the level of reporter gene expression is determined in the presence of the test compound and compared to the level of expression in the absence of the test compound.

To identify compounds that regulate CGRP receptor translation, cells or *in vitro* cell lysates containing CGRP receptor transcripts maybe tested for modulation of CGRP receptor mRNA translation. To assay for inhibitors of CGRP receptor translation, test compounds are assayed for their ability to modulate the translation of CGRP receptor mRNA in *in vitro* translation extracts.

The step of detecting whether a putative regulatory agent binds to a CGRP receptor can be performed by any standard binding assay. Such methods are well known in the art and include competitive binding techniques, equilibrium dialysis or BIAcore methods. In one embodiment, a CGRP receptor of the present invention or a two- or three-dimensional model thereof, is used in a large scale screening of compound libraries and/or in computer-based drug design methods.

The method of identifying a regulatory agent can additionally include detecting whether the putative regulatory agent activates the receptor. Activation of a CGRP receptor can be measured by any suitable method as previously described, including measurement of cAMP increase, calcium mobilization and receptor phosphorylation. Detection of such activities as a result of contact of the receptor with the putative regulatory compound indicates that the compound is a regulator of a CGRP receptor. In one embodiment, the step of detecting whether the putative regulatory compound activates the receptor comprises the steps of contacting the receptor with the agent and detecting whether activation of the receptor is increased in the presence of the putative regulatory compound as compared to in the absence of the putative regulatory compound.

Finally, a putative regulatory compound of the present invention can be evaluated by administering putative regulatory compounds to a non-human test animal and detecting whether the putative regulatory compound reduces AHR in the test animal. Animal models of disease are invaluable to provide evidence to support a hypothesis or justify human experiments. For example, mice have many proteins which share greater than 90% homology with corresponding human proteins. Preferred modes of administration, including dose, route and other aspects of the method are as previously described herein for the therapeutic methods of the present invention. The test animal can be any suitable non-human animal, including any test animal described in the art for evaluation of AHR. The test animal can be, for example, an established mouse model of AHR, as previously described, above and in Takeda et al., (1997). J. Exp. Med. 186, 449-454. Briefly, as an exemplary protocol for this murine model, mice (typically BALB/c) are immunized intraperitoneally with ovalbumin (OVA). The mice are then chronically exposed (*i.e*., challenged) for 8 days (*i.e*., 8 exposures of 30 minutes each in 8 days) to aerosolized OVA. It should be noted that both immunization and subsequent antigen challenge are required to observe a response in mice. To characterize the murine model, pulmonary function measurements of airway resistance (R_{L}) and dynamic compliance (C_{L}) and hyperresponsiveness are obtained as described in Example lbelow.

Compounds identified by any of the above-described methods can be used in a method for the reduction or prevention of AHR as described herein.

The following examples are provided for the purpose of illustration.

### EXAMPLES

### Example 1

This example demonstrates that allergen challenge depletes CGRP in the airways of sensitized mice.

In this experiment and all following experiments described herein, pathogen-free female BALB/c mice were obtained from Jackson Laboratories (Bar Harbor, ME) at 8 wk of age and were maintained on ovalbumin-free diet.

Mice (8/group/experiment) were sensitized by intraperitoneal injection of 20 µg of ovalbumin (Grade V, Sigma) emulsified in 2.25 mg alum (Alum® Inject; Pierce, Rockford, IL) in a total volume of 100 µl on days 0 and 14. On days 28,29 and 30, mice were challenged via the airways by a 20-minute inhalation exposure to aerosols of ovalbumin (1% in saline) obtained from a DeVilbiss ultrasonic nebulizer (particle size 1-5 µm). Age-matched, control animal groups (8/group/experiment) consisted of mice injected with alum alone (non-sensitized) and exposed either to aerosols of saline or to aerosolized ovalbumin, and mice sensitized to ovalbumin but subsequently exposed to aerosols of saline.

For histology and immunohistochemistry, the lungs of the mice were inflated and fixed with 4% paraformaldehyde in PBS for 24 h at 4°C followed by processing into paraffin. Five µm-thick sections were cut from the paraffin blocks, deparaffinized and stained with hematoxylin and eosin for routine histology. Mucus-containing goblet cells were visualized by staining with Periodic Acid Shiff (PAS)-alcian blue.

The pan neuronal marker PGP9.5, CGRP and tissue eosinophils were detected by immunoperoxidase. Unless otherwise stated, all incubations were carried out at room temperature. All washes were performed with TBS (50 mM TRIS-buffered saline pH 7.6) 3 times for 5 min. Endogenous peroxidase was blocked by incubation of tissue sections with H₂O₂ (0.3% in methanol) for 30 minutes. To prevent non-specific binding of conjugated secondary antibody, all sections were preincubated with a non-immune goat serum (5% in TBS) for 30 minutes.

For tissue eosinophils, the sections were pretreated for 5 min with 0.01% trypsin in TBS to retrieve antigens. The primary antibody consisted of a polyclonal rabbit anti-mouse eosinophil MBP optimally diluted 1:3,000 (kindly provided by Dr. J. Lee, Mayo Clinic, Phoenix, AR). A polyclonal rabbit anti-PGP9.5 (Biogenesis Inc., Sandown, NH) was used at a dilution of 1:1000 for specific staining of airway nerve fibers. CGRP was detected with a polyclonal rabbit anti-CGRP antibody (Biogenesis) diluted 1:200. After incubation with the primary antibodies overnight at 4°C, the sections were washed with TBS and incubated for 60 min with a biotinylated goat serum anti-rabbit immunoglobulins (Dako) optimally diluted 1:300. Subsequent steps consisted of a 30-minute incubation with avidin-biotinylated peroxidase complex (ABC, Dako) followed by washes and incubation with a metal-enhanced DAB peroxidase substrate (Pierce). The sections were counterstained with Harris's hematoxylin, dehydrated in graded ethanol, cleared in xylene and mounted with Permount (Fisher). The specificity of CGRP immunostaining was assessed by incubating consecutive tissue sections with anti-CGRP antibody, pre-adsorbed to CGRP (10⁻⁴M), which resulted in complete abolition of the staining.

The data of immunohistochemistry were quantitatively analyzed (morphometric analysis) on a G-3 Macintosh computer using the National Institutes of Health (NIH) Scion Image analysis software. Images of stained lung tissue sections were captured under Olympus BX40 microscope equipped with Kodak MDS 120 digital camera and were transferred to the computer. Images, obtained at the same magnifications as for tissue sections, were also captured from a micrometer scale (1-mm total, subdivisions of 10 µm) and were used for linear calibration of measurements. All non-cartilaginous, intrapulmonary airways were included for the measurements. These airways were divided into central and peripheral airways based on the present inventors' preliminary analyses showing different airway morphometric characteristics (Table 1).

**TABLE 1. Morphometric characteristics of mouse central and peripheral intrapulmonary airways.**

| Airway | Generation | Diameter (mm) | Perimeter (mm) | Muscle layers | Goblet cell* hyperplasia |
|---|---|---|---|---|---|
| Central | 1 st and 2nd | 0.3-0.8 | 1.0-2.5 | 3-5 | Yes |
| Peripheral | 3rd and 4th | <0.3 | <1.0 | 1-2 | No |

| | | | | | |
|---|---|---|---|---|---|
| *In ovalbumin-sensitized and challenged mice (PAS/alcian blue staining). | | | | | |

The number of tissue infiltrating eosinophils was determined by counting all MBP-positive cells present in the airway wall including the adventitia. When eosinophils were present in tissue areas connecting blood vessels to adjacent airways only 50% of these cells were counted for these airways. The density of airway nerve fibers was determined by measuring the surface of PGP9.5-immunoreactive nerve area around the airways. For CGRP, the immunoreactive areas were outlined and the total surface was determined for both of the epithelium and nerve fibers of central and peripheral airways. All measurements were normalized to the length of the basement membrane for the corresponding airways. All measurements were performed on at least 3 serial tissue sections cut from the paraffin blocks at every 50 µm. The measured values were averaged for each animal and the mean values were determined for each group.

For this and all subsequent experiments described herein, the data are presented as the mean values with standard error to the means (SEM) for each group (n=8 per group). Data were analyzed by ANOVA with Tukey's test of multiple comparisons of the means to determine significant differences between the groups. Ap value of 0.05 or less was considered for statistical significance.

The results of this experiment showed that mice sensitized and subsequently exposed to aerosolized ovalbumin developed a characteristic peribronchial and perivascular tissue eosinophilic inflammation associated with a marked goblet cell hyperplasia and mucus production (data not shown). Such histopathological changes were not seen in the lungs of control mice. Immunostaining for the pan neuronal marker PGP9.5 revealed no differences in the overall density of airway nerve fibers between control mice and sensitized and challenged mice. However, there was a significant depletion of CGRP from the bronchial epithelium and submucosal nerve plexuses in intrapulmonary airways of ovalbumin-sensitized and challenged mice. In control mice, CGRP was normally expressed in neuroepithelial bodies localized most frequently at the branching points of central intrapulmonary airways with lesser expression in the epithelium of peripheral airways. CGRP-positive nerve fibers were detected in the submucosal area of normal airways showing intimate contact with neuroepithelial bodies and smooth muscle bundles.

Table 2 summarizes the results of morphometric analyses showing significant changes in tissue expression of CGRP in the lungs of sensitized and challenged animals. The results demonstrate that both sensitization and allergen challenge were required for the depletion of CGRP. Indeed, the expression of CGRP remained unchanged in the lungs of non-sensitized mice that were exposed to aerosolized ovalbumin or saline, and in mice that were sensitized to ovalbumin but subsequently exposed to saline.

**TABLE 2. Effect of sensitization and ovalbumin challenge on CGRP immunoreactivity and nerve fiber density in mouse intrapulmonary airways. The data are presented as mean ± SEM (n=8) immunoreactive tissue area normalized to the length of basement membrane (µm²/mm).**

| | Non-sensitized + Saline | Non-sensitized + Avalbumin | Sensitized + Saline | Sensitized Ovalbumin |
|---|---|---|---|---|
| **CGRP** | | | | |
| Central Airways | | | | |
| Epithelium | 212 ± 41^{a} | 21 ± 38^{a} | 182 ± 22^{a} | 35 ± 12^{b} |
| Nerves | 43 ± 8^{a} | 40 ± 5^{a} | 37 ±10^{a} | 5 ± 1^{b} |
| Peripheral Airways | | | | |
| Epithelium | 2.8 ±0.5^{a} | 3.0 ± 0.4^{a} | 2.5 ± 0.7^{a} | 0^{b} |
| Nerves | 2.8 ± 0.9^{a} | 3.3 ± 0.8^{a} | 2.0 ± 0.7^{a} | 0^{b} |
| **PGP9.5** | | | | |
| Central Airways | 2152 ± 326^{a} | 2384 ± 395^{a} | 2391 ± 273^{a} | 2308 ± 487^{a} |
| Peripheral Airways | 312 ± 76^{a} | 302 ± 62^{a} | 283 ± 32^{a} | 297 ± 47^{a} |

Values labeled with the same letter are not statistically different.

### Example 2

This example demonstrates that allergen-mediated CGRP depletion is dependent on the development of eosinophilic airway inflammation in sensitized mice.

To determine if the depletion of CGRP that occurs following sensitization and allergen exposure is dependent on the development of eosinophilic airway inflammation, the effects of treatments with anti-VLA4 and anti-IL5 antibodies on the expression of this neuropeptide were examined in ovalbumin-sensitized and challenged mice. For this experiment, the rat anti-mouse Very Late Antigen (VLA)-4 and anti-mouse IL-5 monoclonal antibodies were purified, respectively, from cultures of the hybridoma cell lines PS/2 and TRFK-5 under endotoxin-free conditions using a protein G-sepharose gel affinity column (Pharmacia, Uppsala, Sweden). The hybridoma cell lines were obtained from American Type Culture Collection (Manassas, VA). Non-immune rat IgG (Sigma) was used as control antibody. Mice were treated by a single intravenous injection of anti-VLA-4, anti-IL5 or rat IgG (2 mg/kg) 2h prior to the first ovalbumin aerosol challenge.

Briefly, mice were sensitized and challenged to ovalbumin as described in Example 1. Airway function was assessed in vivo in anesthetized, mechanically ventilated mice as previously described (see, for example, Takeda et al., 1997, J. Exp. Med. 186, 449-454) by measuring changes in lung resistance (R_{L}) and dynamic compliance (Cdyn) in response to intratracheal challenge with aerosolized methacholine at doses of 1.56, 3.125, 6.25 and 12.5 mg/ml in saline. Baseline values were recorded from data obtained after intra-tracheal challenge with aerosolized saline. The data are presented in percent of change from baseline R_{L} and Cdyn.

Following assessment of airway function, the lungs were lavaged once with 1 ml of sterile Hank's balanced salt solution (HBSS) pre-warmed at 37°C. The recovered BAL fluids were placed in Eppendorf tubes and were centrifuged at 4°C for 5 min at 1,500 rpm. The obtained cell pellets were resuspended in 200 µl of sterile phosphate-buffered saline (PBS) and total cell numbers were determined from counting of crystal violet-stained aliquots using a hemacytometer. Differential cell counts were determined from cytospin preparations stained with Leukostat (Fisher Diagnostics, Pittsburgh, PA). At least 200 cells were counted from each slide in a blinded fashion.

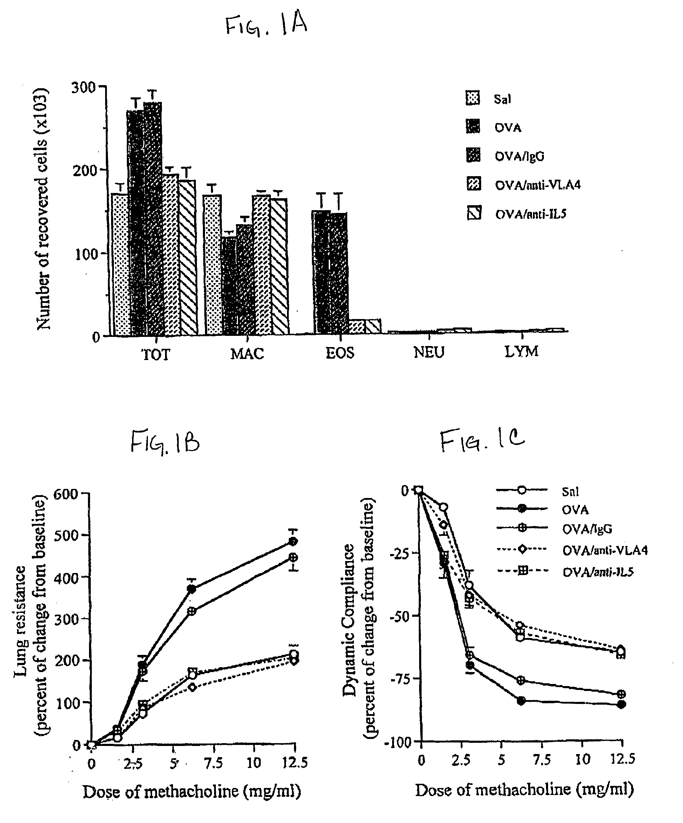

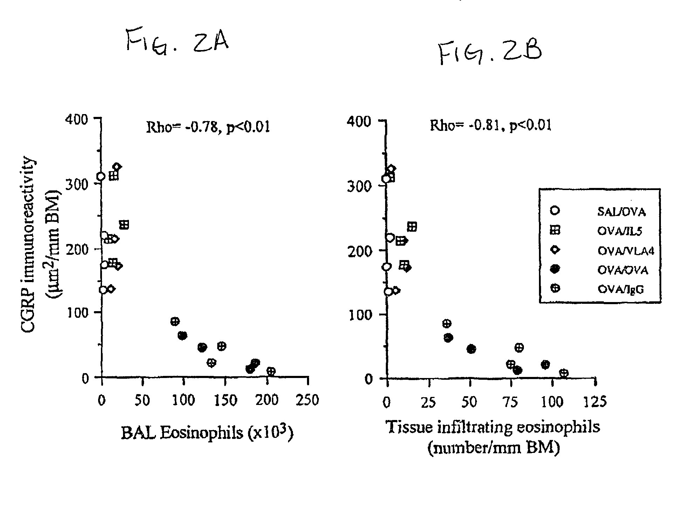

Treatment of sensitized mice with anti-VLA-4 and anti-IL5 antibodies prior to allergen challenge markedly reduced the number of eosinophils in the BAL (Fig. 1A) and completely abolished the development of AHR in these animals (Figs. 1B-1C). These treatments also considerably reduced the numbers of tissue infiltrating eosinophils and prevented the allergen-mediated depletion of CGRP in sensitized mice (Table 3). Further, an overall negative correlation was observed between CGRP immunoreactivity (*i.e*., the ability to detect CGRP by antibody) in central intrapulmonary airways and the numbers of tissue infiltrating eosinophils (Fig. 2B) as well as BAL eosinophils (Fig. 2A).

**TABLE 3. Effect of anti-VLA4 and anti-IL5 treatments on tissue infiltrating eosinophils and CGRP immunoreactivity in central intrapulmonary mouse airways. Data are means ± SEM (n=8).**

| | Saline | OVA/aIL5 | OVA/aVLA4 | OVA/IgG |
|---|---|---|---|---|
| **Eosinophils** (cells/mm BM) | 1 ± 0^{a} | 9 ± 3^{a} | 8 ± 2^{a} | 75 ± 15^{b} |
| **CGRP** (µm²/mm BM) | | | | |
| Epithelium | 196 ± 20^{a} | 235 ± 28^{a} | 213 ± 4^{a} | 41 ± 17^{b} |
| Nerves | 44 ± 11^{a} | 45 ± 13^{a} | 45 ± 10^{a} | 2 ± 1^{b} |

| | | | | |
|---|---|---|---|---|
| Values labeled with the same letter are not statistically different | | | | |

### Example 3

This example demonstrates that allergen-induced airway hyperresponsiveness is abolished by CGRP in sensitized mice.

To determine the role of CGRP in allergen-induced AHR, the effects of two pharmacological approaches were examined: (1)- administration of CGRP(8-37) to antagonize the effect of endogenous CGRP, and (2)- administration of exogenous CGRP to compensate for the in vivo depletion. The human synthetic calcitonin gene-related peptide (α-CGRP) and the highly selective CGRP receptor antagonist, human synthetic CGRP (fragment 8-37), were obtained from Sigma Chemical Co. (St. Louis, MO) and were dissolved in endotoxin-free, non-pyrogenic saline. In this experiment, mice were treated by intraperitoneal injection of CGRP (20 µg/kg) or CGRP fragment 8-37 (100 µg/kg). Sensitization, challenge, and evaluation of BAL and AHR in the mice were performed as described in Examples 1 and 2.

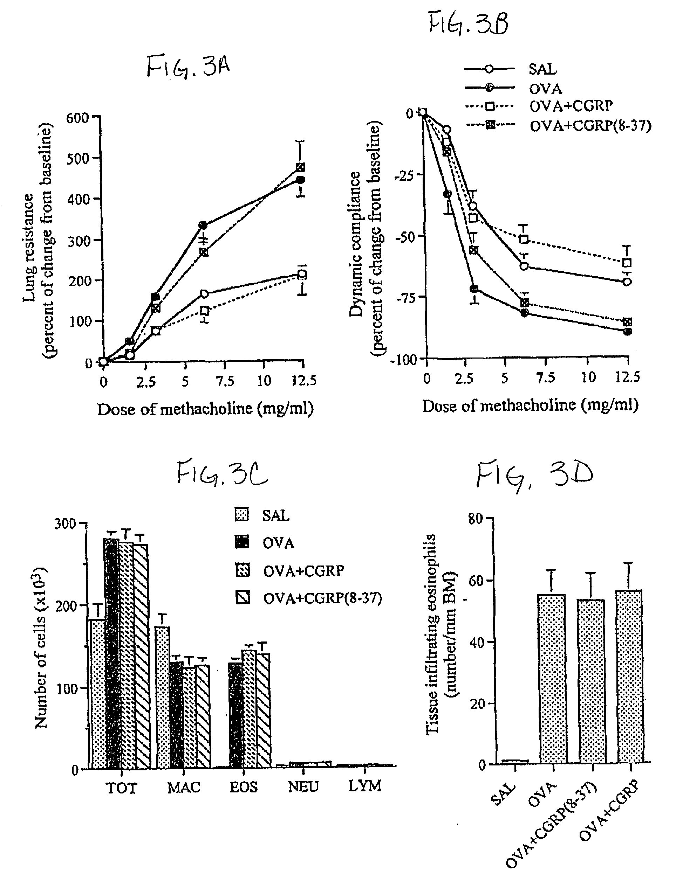

The results showed that treatment of mice with CGRP(8-37), at 2h prior to each allergen challenge, did notproduce any significant change in the extent of measured AHR (Figs. 3A and 3B). By contrast, similar treatment of mice with exogenous CGRP completely suppressed the development of AHR in sensitized animals. Both treatments had no significant effect on the numbers of BAL (Fig. 3C) and tissue infiltrating (Fig. 3D) eosinophils.

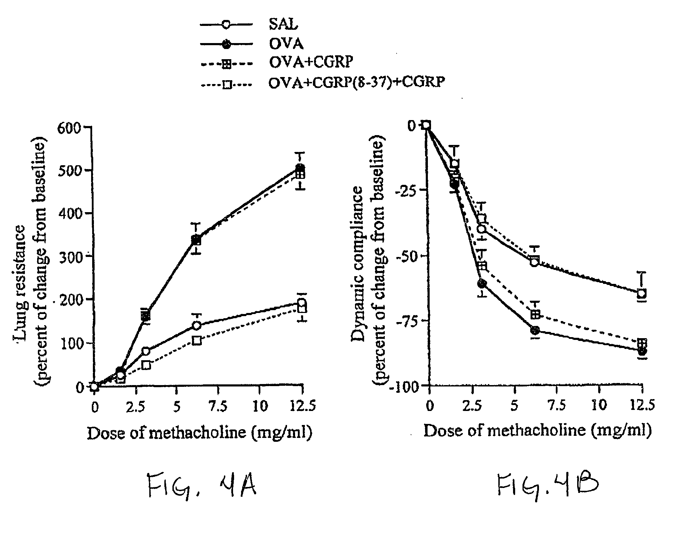

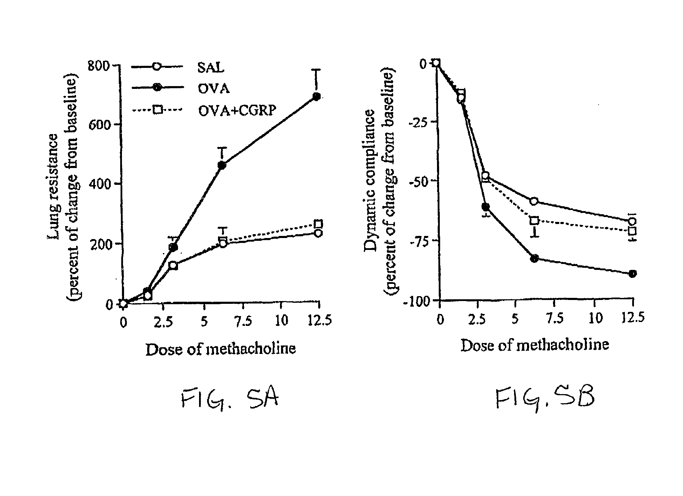

Intraperitoneal administration of exogenous CGRP (α-CGRP) after the period of allergen challenge, at 2 h prior to the assessment of airway function, also abolished AHR in sensitized mice. Importantly, this inhibitory effect of CGRP was totally neutralized by pretreatment of the animals with the receptor antagonist CGRP(8-37) (Figs. 4A and 4B). AHR was also abolished in sensitized and challenged mice that were exposed for 15 minutes to aerosolized CGRP (10⁻⁶ M) at 2 h before assessment of airway function (Figs. 5A and 5B).

The experiments described in Examples 1-3 above were undertaken to determine the role of CGRP in the development of airway hyperresponsiveness in a mouse model of ovalbumin-induced allergic airway inflammation. The results demonstrated that allergen exposure depletes CGRP from the bronchial epithelium and submucosal nerve fibers without altering the overall density of nerve fibers in the airways of sensitized mice. This depletion required both sensitization and allergen challenge and was dependent on the development of airway eosinophilic inflammation since it correlated with the number of BAL and tissue infiltrating eosinophils and was prevented by treatments that reduced the recruitment of these cells into the airways. Without being bound by theory, the present inventors believe that since allergen challenge depleted CGRP from sensitized mouse airways, this neuropeptide is released in response to inflammatory mediators produced by activated eosinophils to modulate allergic airway hyperresponsiveness in this animal model.

In summary, the present study demonstrated that allergen exposure depletes CGRP from the airways of sensitized mice in an eosinophil-dependent manner that contributes to the development of an exaggerated bronchoconstriction to methacholine and when present, CGRP can restore normal airway tone. These findings demonstrate that a deficit in the airway content of CGRP subsequent to an allergen exposure may be an important mechanism that contributes to the development of airway hyperresponsiveness in allergic asthma, as well as other conditions characterized by airway hyperresponsiveness, and additionally show that administration of CGRP reduces airway hyperresponsiveness.

## Claims

1. The use of an agent that binds to and activates a calcitonin gene related peptide (CGRP) receptor in the lungs of a mammal in the manufacture of a medicament for treatment of allergen-induced airway hyperresponsiveness in allergy, asthma and other respiratory conditions selected from the group consisting of chronic obstructive pulmonary disease, allergic bronchopulmonary aspergillosis, hypersensitivity pneumonia, eosinophilic pneumonia, emphysema, bronchitis, allergic bronchitis bronchiectasis, cystic fibrosis, tuberculosis, hypersensitivity pneumonitis, occupational asthma, sarcoid, reactive airway disease syndrome, interstitial lung disease, hyper-eosinophilic syndrome, rhinitis, sinusitis, exercise-induced asthma, pollution-induced asthma, cough variant asthma, or parasitic lung disease; wherein the agent is selected from the group consisting of: CGRP, a fragment of CGRP that binds to and activates a CGRP receptor, an agonist homologue of CGRP that binds to and activates a CGRP receptor, and an antibody or antigen-binding fragment thereof that selectively binds to and activates a CGRP receptor.

2. The use according to claim 1, wherein the mammal has been sensitized to an allergen and has been exposed to, or is at risk of being exposed to, an amount of the allergen that is sufficient to induce allergen-induced airway hyperresponsiveness in the mammal in the absence of the agent.

3. The use according to any one of the preceding claims wherein the medicament comprises an amount of the agent determinable by monitoring the mammal to detect whether allergen-induced airway hyperresponsiveness in the mammal is inhibited, wherein if allergen-induced airway hyperresponsiveness is detected in the mammal, additional amounts of the agent are administered until allergen-induced airway hyperresponsiveness is not detected in the mammal.

4. The use according to any one of Claims 1 to 3, wherein the medicament is for administration within a time period of between 48 hours or less prior to exposure to an airway hyperresponsiveness provoking stimulus that is sufficient to induce allergen-induced airway hyperresponsiveness, and within 48 hours or less after the detection of the first symptoms of allergen-induced airway hyperresponsiveness.

5. The use according to any one of claims 1 to 3, wherein the medicament is for administration upon the detection of the first symptoms of allergen-induced airway hyperresponsiveness.

6. The use according to any one of claims 1 to 3, wherein the medicament is for administration within 1 hour after the detection of the first symptoms of allergen-induced airway hyperresponsiveness.

7. The use according to any one of claims 1 to 3, wherein the medicament is for administration within 12 hours or less prior to exposure to a airway hyperresponsiveness provoking stimulus that is sufficient to induce allergen-induced airway hyperresponsiveness.

8. The use according to any one of claims 1 to 3, wherein the medicament is for administration within 2 hours or less prior to exposure to a airway hyperresponsiveness provoking stimulus that is sufficient to induce allergen-induced AHR.

9. The use according to any one of claims 1 to 3, wherein the medicament is for administration to the mammal every one to two days.

10. The use according to claim 1, wherein the antibody is a divalent antibody.

11. The use according to claim 1, wherein the antibody is a bi-specific antibody, wherein the antibody selectively binds to the CGRP receptor and to an antigen on a cell selected from the group consisting of a lung smooth muscle cell and a lung epithelial cell.

12. The use according to any one of the preceding claims, wherein the medicament is for administration at a dose of from 0.1 µg x kilogram⁻¹ to 20 µg x kilogram⁻¹ body weight of the mammal.

13. The use according to any one of the preceding claims, wherein the medicament is for administration at a dose of from 0.1 µg x kilogram⁻¹ to 10µg x kilogram⁻¹ body weight of the mammal.

14. The use according to any one of the preceding claims, wherein the medicament is for administration at a dose of from 0.1 µg x kilogram⁻¹ to 5 µg x kilogram⁻¹ body weight of the mammal.

15. The use according to any one of the preceding claims, wherein the medicament is for targeting the agent to cells in the lung of the mammal selected from the group consisting of smooth muscle cells and epithelial cells.

16. The use according to any one of the preceding claims, wherein the medicament is for direct delivery of the agent to the lung of the mammal.

17. The use according to any one of the preceding claims, wherein the medicament is for administration by aerosol delivery.

18. The use according to any one of the preceding claims, wherein the medicament is for administration by parenteral delivery.

19. The use according to any one of the preceding claims, wherein the medicament is for administration by oral delivery.

20. The use according to any one of the preceding claims, wherein the medicament further comprises another agent selected from the group consisting of: corticosteroids (oral, inhaled and injected), β -agonists (long or short acting), leukotriene modifiers (inhibitors or receptor antagonists), antihistamines, phosphodiesterase inhibitors, sodium cromoglycate, nedocrimal, and theophylline.

21. The use according to any one of the preceding claims, wherein the medicament further comprises a CGRP receptor activity modifying protein (RAMP).

22. The use according to any one of the preceding claims, wherein the medicament further comprises a pharmaceutically acceptable excipient.

23. The use according to any one of the preceding claims, wherein the mammal is a human.

24. The use according to any one of the preceding claims, wherein the agent is CORP.

25. The use according to any one of the preceding claims, wherein the agent is α -CGRP.

26. A method to identify an agent for reducing allergen-induced airway hyperresponsiveness in a mammal, comprising:
a) contacting a calcitonin gene related peptide (CGRP) receptor with a putative regulatory agent;
b) detecting whether the putative regulatory agent binds to the CGRP receptor;
c) administering a putative regulatory agent which binds to the CGRP receptor to a non-human test mammal in which allergen-induced airway hyperresponsiveness can be induced and detecting whether the putative regulatory agent reduces allergen-induced airway hyperresponsiveness in the test mammal upon induction of allergen-induced airway hyperresponsiveness in the presence of the putative regulatory agent as compared to in the absence of the putative regulatory agent;
wherein putative regulatory agents that bind to the CGRP receptor and that reduce allergen-induced airway hyperresponsiveness in the test mammal are identified as agents which reduce allergen-induced airway hyperresponsiveness.

27. The method according to claim 26, wherein the step (c) of administering comprises administering the putative regulatory agent which binds to the CGRP receptor to a non-human test mammal that has been sensitized to an allergen and detecting whether the putative regulatory agent reduces allergen-induced airway hyperresponsiveness in the test mammal when the mammal is challenged with the allergen, as compared to in the absence of the putative regulatory agent;
wherein putative regulatory agents that bind to the CGRP receptor and that reduce airway hyperresponsiveness in the test mammal are identified as agents which reduce allergen-induced airway hyperresponsiveness.

28. The method according to any of claims 26 to 27, wherein the CGRP receptor is a soluble receptor.

29. The method according to any of claims 26 to 28, wherein in part (a), the CGRP receptor is expressed by a cell, and wherein the step (b) of detecting further comprises detecting whether the CGRP receptor is activated by the putative regulatory compound.

30. The method according to any of claims 26 to 29, wherein the non-human test mammal is a mouse.

31. The method according to any of claims 26 to 30, wherein the putative regulatory agent is a product of rational drug design.

32. The method according to any of claims 26 to 31, wherein the putative regulatory agent is an antibody.

## Patentansprüche

1. Verwendung eines Agens, das sich an einen CGRP-(Calcitonin Gene-Related Peptide)-Rezeptor in der Lunge eines Säugetieres bindet und ihn aktiviert, bei der Herstellung eines Medikaments zur Behandlung von allergeninduzierter Atemwegshyperreaktivität bei Allergie, Asthma und anderen Atemwegserkrankungen, die ausgewählt sind aus der Gruppe bestehend aus chronisch obstruktiver Lungenerkrankung, allergischer bronchopulmonaler Aspergillose, Hypersensibilitätspneumonie, eosinophiler Pneumonie, Emphysem, Bronchitis, allergischer Bronchitis, Bronchiektase, zystischer Fibrose, Tuberkulose, Hypersensibilitätspneumonitis, Berufsasthma, Sarkoid, reaktivem Atemwegserkrankungssyndrom, interstitieller Lungenkrankheit, hypereosinophilem Syndrom, Rhinitis, Sinusitis, Belastungsasthma, durch Umweltverschmutzung induziertem Asthma, Cough-variant Asthma oder parasitärer Lungenkrankheit; wobei das Agens ausgewählt ist aus der Gruppe bestehend aus: CGRP, einem Fragment von CGRP, das sich an einen CGRP-Rezeptor bindet und ihn aktiviert, einem agonistischen Homolog von CGRP, das sich an einen CGRP-Rezeptor bindet und ihn aktiviert, und einem Antikörper oder Antigenbindungsfragment davon, der/das sich selektiv an einen CGRP-Rezeptor bindet und ihn aktiviert.

2. Verwendung nach Anspruch 1, wobei das Säugetier für ein Allergen sensibilisiert und einer Menge des Allergens ausgesetzt wurde oder bei dem die Gefahr einer solchen Aussetzung besteht, die ausreicht, um eine allergeninduzierte Atemwegshyperreaktivität in dem Säugetier in Abwesenheit des Agens auszulösen.

3. Verwendung nach einem der vorherigen Ansprüche, wobei das Medikament eine Menge des Agens beinhaltet, die durch Beobachten des Säugetiers bestimmbar ist, um zu ermitteln, ob eine allergeninduzierte Atemwegshyperreaktivität in dem Säugetier inhibiert wird, wobei, wenn allergeninduzierte Atemwegshyperreaktivität in dem Säugetier ermittelt wird, zusätzliche Mengen des Agens verabreicht werden, bis keine allergeninduzierte Atemwegshyperreaktivität in dem Säugetier ermittelt wird.

4. Verwendung nach einem der Ansprüche 1 bis 3, wobei das Medikament für eine Verabreichung innerhalb eines Zeitraums von 48 Stunden oder weniger vor der Aussetzung gegenüber einem Atemwegshyperreaktivität auslösenden Reiz, der ausreicht, um eine allergeninduzierte Atemwegshyperreaktivität zu induzieren, und innerhalb von 48 Stunden oder weniger nach dem Auftreten der ersten Symptome einer allergeninduzierten Atemswegshyperreaktivität vorgesehen ist.

5. Verwendung nach einem der Ansprüche 1 bis 3, wobei das Medikament für eine Verabreichung nach dem Ermitteln der ersten Symptome einer allergeninduzierten Atemwegshyperreaktivität vorgesehen ist.

6. Verwendung nach einem der Ansprüche 1 bis 3, wobei das Medikament für eine Verabreichung innerhalb von 1 Stunde nach dem Auftreten der ersten Symptome einer allergeninduzierten Atemwegsreaktivität vorgesehen ist.

7. Verwendung nach einem der Ansprüche 1 bis 3, wobei das Medikament für eine Verabreichung innerhalb von 12 Stunden oder weniger vor der Aussetzung gegenüber einem Atemwegshyperreaktivität auslösenden Reiz vorgesehen ist, der ausreicht, um eine allergeninduzierte Atemswegshyperreaktivität zu induzieren.

8. Verwendung nach einem der Ansprüche 1 bis 3, wobei das Medikament für eine Verabreichung innerhalb von 2 Stunden oder weniger vor der Aussetzung gegenüber einem Atemwegshyperreaktivität auslösenden Reiz vorgesehen ist, der ausreicht, um eine allergeninduzierte AHR auszulösen.

9. Verwendung nach einem der Ansprüche 1 bis 3, wobei das Medikament für eine Verabreichung an das Säugetier alle ein bis zwei Tage vorgesehen ist.

10. Verwendung nach Anspruch 1, wobei der Antikörper ein bivalenter Antikörper ist.

11. Verwendung nach Anspruch 1, wobei der Antikörper ein bispezifischer Antikörper ist, wobei sich der Antikörper selektiv an einen CGRP-Rezeptor und an ein Antigen auf einer Zelle bindet, die aus der Gruppe bestehend aus einer glatten Muskelzelle der Lunge und einer Lungenepithelzelle ausgewählt ist.

12. Verwendung nach einem der vorherigen Ansprüche, wobei das Medikament für eine Verabreichung in einer Dosis von 0,1 µg x Kilogramm bis 20 µg x Kilogramm⁻¹ Körpergewicht des Säugetiers vorgesehen ist.

13. Verwendung nach einem der vorherigen Ansprüche, wobei das Medikament zur Verabreichung in einer Dosis von 0,1 µg x Kilogramm⁻¹ bis 10 µg x Kilogramm⁻¹ Körpergewicht des Säugetiers vorgesehen ist.

14. Verwendung nach einem der vorherigen Ansprüche, wobei das Medikament zur Verabreichung in einer Dosis von 0,1 µg x Kilogramm⁻¹ bis 5 µg x Kilogramm⁻¹ Körpergewicht des Säugetiers vorgesehen ist.

15. Verwendung nach einem der vorherigen Ansprüche, wobei das Medikament zum Targetieren des Agens auf Zellen in der Lunge des Säugetiers vorgesehen ist, die aus der Gruppe bestehend aus glatten Muskelzellen und Epithelzellen ausgewählt sind.

16. Verwendung nach einem der vorherigen Ansprüche, wobei das Medikament für eine direkte Zuführung des Agens zur Lunge des Säugetiers vorgesehen ist.

17. Verwendung nach einem der vorherigen Ansprüche, wobei das Medikament zur Verabreichung durch Aerosolzuführung vorgesehen ist.

18. Verwendung nach einem der vorherigen Ansprüche, wobei das Medikament zur Verabreichung durch parenterale Zuführung vorgesehen ist.

19. Verwendung nach einem der vorherigen Ansprüche, wobei das Medikament zur Verabreichung durch orale Zuführung vorgesehen ist.

20. Verwendung nach einem der vorherigen Ansprüche, wobei das Medikament ferner ein weiteres Agens beinhaltet, das ausgewählt ist aus der Gruppe bestehend aus:
Corticosteroide (oral, inhaliert und injiziert), β-Agonisten (lang- und kurzwirkend), Leukotrienmodifikatoren (Inhibitoren oder Rezeptorantagonisten), Antihistamine, Phosphodiesteraseinhibitoren, Natriumcromoglykat, Nedocrimal und Theophyllin.

21. Verwendung nach einem der vorherigen Ansprüche, wobei das Medikament ferner ein CGRP-Rezeptoraktivitäts-modifizierendes Protein (RAMP) beinhaltet.

22. Verwendung nach einem der vorherigen Ansprüche, wobei das Medikament ferner einen pharmazeutisch akzeptablen Exzipienten beinhaltet.

23. Verwendung nach einem der vorherigen Ansprüche, wobei das Säugetier ein Mensch ist.

24. Verwendung nach einem der vorherigen Ansprüche, wobei das Agens CGRP ist.

25. Verwendung nach einem der vorherigen Ansprüche, wobei das Agens α-CGRP ist.

26. Verfahren zum Identifizieren eines Agens zum Reduzieren von allergeninduzierter Atemwegshyperreaktivität in einem Säugetier, das Folgendes beinhaltet:
a) Inkontaktbringen eines CGRP-(Calcitonin Gene-Related Peptide)-Rezeptors mit einem vermuteten regulatorischen Agens;
b) Ermitteln, ob sich das vermutete regulatorische Agens an den CGRP-Rezeptor bindet;
c) Verabreichen eines vermuteten regulatorischen Agens, das sich an den CGRP-Rezeptor bindet, an ein nicht menschliches Versuchssäugetier, in dem allergeninduzierte Atemwegshyperreaktivität induziert werden kann, und Ermitteln, ob das vermutete regulatorische Agens die allergeninduzierte Atemswegshyperreaktivität in dem Versuchssäugetier nach dem Induzieren der allergeninduzierten Atemswegshyperreaktivität in Anwesenheit des vermuteten regulatorischen Agens im Vergleich zur Abwesenheit des vermuteten regulatorischen Agens reduziert;
wobei vermutete regulatorische Agenzien, die sich an den CGRP-Rezeptor binden und die allergeninduzierte Atemwegshyperreaktivität in dem Testsäugetier reduzieren, als Agenzien identifiziert werden, die eine allergeninduzierte Atemwegshyperreaktivität reduzieren.

27. Verfahren nach Anspruch 26, wobei der Verabreichungsschritt (c) Folgendes beinhaltet:
Verabreichen des vermuteten regulatorischen Agens, das sich an den CGRP-Rezeptor bindet, an ein nicht menschliches Versuchssäugetier, das gegenüber einem Allergen sensibilisiert wurde, und Ermitteln, ob das vermutete regulatorische Agens allergeninduzierte Atemwegshyperreaktivität in dem Versuchssäugetier im Vergleich zur Abwesenheit des vermuteten regulatorischen Agens reduziert, wenn das Säugetier mit dem Allergen provoziert wird;
wobei vermutete regulatorische Agenzien, die sich an den CGRP-Rezeptor binden und die Atemwegshyperreaktivität in dem Versuchssäugetier reduzieren, als Agenzien identifiziert werden, die allergeninduzierte Atemwegshyperreaktivität reduzieren.

28. Verfahren nach einem der Ansprüche 26 bis 27, wobei der CGRP-Rezeptor ein löslicher Rezeptor ist.

29. Verfahren nach einem der Ansprüche 26 bis 28, wobei in Teil (a) der CGRP-Rezeptor durch eine Zelle exprimiert wird und wobei der Ermittlungsschritt (b) ferner das Ermitteln beinhaltet, ob der CGRP-Rezeptor durch die vermutete regulatorische Verbindung aktiviert wird.

30. Verfahren nach einem der Ansprüche 26 bis 29, wobei das nicht menschliche Versuchssäugetier eine Maus ist.

31. Verfahren nach einem der Ansprüche 26 bis 30, wobei das vermutete regulatorische Agens ein Produkt rationalen Wirkstoffdesigns ist.

32. Verfahren nach einem der Ansprüche 26 bis 31, wobei das vermutete regulatorische Agens ein Antikörper ist.

## Revendications

1. Utilisation d'un agent qui se lie à et active un récepteur du peptide lié au gène de la calcitonine (CGRP) dans les poumons d'un mammifère dans la fabrication d'un médicament pour le traitement de l'hyperréactivité des voies aériennes induite par un allergène associée à une allergie, un asthme et d'autres affections respiratoires sélectionnées dans le groupe consistant en la bronchopneumopathie chronique obstructive, l'aspergillose bronchopulmonaire allergique, la pneumopathie d'hypersensibilité, la pneumonie à éosinophiles, l'emphysème, la bronchite, la bronchite allergique et la bronchiectasie, la fibrose kystique, la tuberculose, la pneumonite d'hypersensibilité, l'asthme professionnel, la sarcoïdose, le syndrome de dysfonction réactive des bronches, la pneumopathie interstitielle, le syndrome hyperéosinophilique, la rhinite, la sinusite, l'asthme d'effort, l'asthme induit par la pollution, l'asthme tussif ou les parasitoses pulmonaires, dans laquelle l'agent est sélectionné dans le groupe consistant en les suivants : CGRP, fragment du CGRP qui se lie à et active un récepteur du CGRP, agoniste homologue du CGRP qui se lie à et active un récepteur du CGRP et anticorps ou fragment se liant à l'antigène de celui-ci qui se lie sélectivement à et active un récepteur du CGRP.

2. Utilisation selon la revendication 1, dans laquelle le mammifère a été sensibilisé à un allergène et a été exposé, ou est à risque d'être exposé, à une quantité de l'allergène qui est suffisante pour déclencher une hyperréactivité des voies aériennes induite par l'allergène chez le mammifère en l'absence de l'agent.

3. Utilisation selon l'une quelconque des revendications précédentes, dans laquelle le médicament comprend l'agent en une quantité déterminable par un suivi du mammifère permettant de détecter si l'hyperréactivité des voies aériennes induite par l'allergène chez le mammifère est inhibée et dans laquelle des quantités additionnelles de l'agent sont administrées si une hyperréactivité des voies aériennes induite par l'allergène est détectée chez le mammifère jusqu'à résolution de l'hyperréactivité des voies aériennes induite par l'allergène chez le mammifère.

4. Utilisation selon l'une quelconque des revendications 1 à 3, dans laquelle le médicament est conçu pour être administré dans les 48 heures ou moins qui précèdent une exposition à un stimulus provoquant une hyperréactivité des voies aériennes qui est suffisante pour déclencher une hyperréactivité des voies aériennes induite par un allergène et dans les 48 heures ou moins qui suivent la détection des premiers symptômes d'hyperréactivité des voies aériennes induite par un allergène.

5. Utilisation selon l'une quelconque des revendications 1 à 3, dans laquelle le médicament est conçu pour être administré à la détection des premiers symptômes d'hyperréactivité des voies aériennes induite par un allergène.

6. Utilisation selon l'une quelconque des revendications 1 à 3, dans laquelle le médicament est conçu pour être administré dans l'heure qui suit la détection des premiers symptômes d'hyperréactivité des voies aériennes induite par un allergène.

7. Utilisation selon l'une quelconque des revendications 1 à 3, dans laquelle le médicament est conçu pour être administré dans les 12 heures ou moins qui précèdent une exposition à un stimulus provoquant une hyperréactivité des voies aériennes qui est suffisante pour déclencher une hyperréactivité des voies aériennes induite par un allergène.

8. Utilisation selon l'une quelconque des revendications 1 à 3, dans laquelle le médicament est conçu pour être administré dans les 2 heures ou moins qui précèdent une exposition à un stimulus provoquant une hyperréactivité des voies aériennes qui est suffisante pour déclencher une hyperréactivité des voies aériennes induite par un allergène.

9. Utilisation selon l'une quelconque des revendications 1 à 3, dans laquelle le médicament est conçu pour être administré au mammifère tous les un à deux jours.

10. Utilisation selon la revendication 1, dans laquelle l'anticorps est un anticorps bivalent.

11. Utilisation selon la revendication 1, dans laquelle l'anticorps est un anticorps bispécifique et dans laquelle l'anticorps se lie sélectivement au récepteur du CGRP et à un antigène sur une cellule sélectionnée dans le groupe consistant en une cellule musculaire lisse du poumon et une cellule épithéliale du poumon.

12. Utilisation selon l'une quelconque des revendications précédentes, dans laquelle le médicament est conçu pour être administré à une dose allant de 0,1 µg x kilogramme⁻¹ à 20 µg x kilogramme⁻¹ de poids corporel du mammifère.

13. Utilisation selon l'une quelconque des revendications précédentes, dans laquelle le médicament est conçu pour être administré à une dose allant de 0,1 µg x kilogramme⁻¹ à 10 µg x kilogramme⁻¹ de poids corporel du mammifère.

14. Utilisation selon l'une quelconque des revendications précédentes, dans laquelle le médicament est conçu pour être administré à une dose allant de 0,1 µg x kilogramme⁻¹ à 5 µg x kilogramme⁻¹ de poids corporel du mammifère.

15. Utilisation selon l'une quelconque des revendications précédentes, dans laquelle le médicament est conçu pour cibler des cellules du poumon du mammifère avec l'agent, lesdites cellules étant sélectionnées dans le groupe consistant en des cellules musculaires lisses et des cellules épithéliales.

16. Utilisation selon l'une quelconque des revendications précédentes, dans laquelle le médicament est conçu pour délivrer directement l'agent au poumon du mammifère.

17. Utilisation selon l'une quelconque des revendications précédentes, dans laquelle le médicament est conçu pour être administré par voie inhalée.

18. Utilisation selon l'une quelconque des revendications précédentes, dans laquelle le médicament est conçu pour être administré par voie parentérale.

19. Utilisation selon l'une quelconque des revendications précédentes, dans laquelle le médicament est conçu pour être administré par voie orale.

20. Utilisation selon l'une quelconque des revendications précédentes, dans laquelle le médicament comprend également un autre agent sélectionné dans le groupe consistant en les suivants : corticostéroïdes (oraux, inhalés ou injectables), β-agonistes (à durée d'action prolongée ou courte), modificateurs des leucotriènes (inhibiteurs ou antagonistes des récepteurs des leucotriènes), antihistaminiques, inhibiteurs des phosphodiestérases, cromoglycate de sodium, nédocrimal et théophylline.

21. Utilisation selon l'une quelconque des revendications précédentes, dans laquelle le médicament comprend également une protéine modifiant l'activité du récepteur (RAMP) du CGRP.

22. Utilisation selon l'une quelconque des revendications précédentes, dans laquelle le médicament comprend également un excipient pharmaceutiquement acceptable.

23. Utilisation selon l'une quelconque des revendications précédentes, dans laquelle le mammifère est humain.

24. Utilisation selon l'une quelconque des revendications précédentes, dans laquelle l'agent est le CGRP.

25. Utilisation selon l'une quelconque des revendications précédentes, dans laquelle l'agent est le CGRP-α.

26. Méthode d'identification d'un agent capable de réduire l'hyperréactivité des voies aériennes induite par un allergène chez un mammifère, qui comprend les étapes consistant à :
a) mettre en contact un récepteur du peptide lié au gène de la calcitonine (CGRP) avec un agent régulateur putatif ;
b) détecter si l'agent régulateur putatif se lie au récepteur du CGRP ;
c) administrer un agent régulateur putatif qui se lie au récepteur du CGRP à un mammifère test non humain chez lequel une hyperréactivité des voies aériennes induite par un allergène peut être provoquée et détecter si l'agent régulateur putatif réduit l'hyperréactivité des voies aériennes induite par l'allergène chez le mammifère test au déclenchement d'une hyperréactivité des voies aériennes induite par l'allergène en la présence de l'agent régulateur putatif par comparaison à ce qui est observé en son absence ;
dans laquelle les agents régulateurs putatifs qui se lient au récepteur du CGRP et réduisent l'hyperréactivité des voies aériennes induite par un allergène chez le mammifère test sont identifiés comme des agents qui réduisent l'hyperréactivité des voies aériennes induite par l'allergène.

27. Méthode selon la revendication 26, dans laquelle l'étape d'administration (c) consiste à administrer l'agent régulateur putatif qui se lie au récepteur du CGRP à un mammifère test non humain qui a été sensibilisé à un allergène et à détecter si l'agent régulateur putatif réduit ou non l'hyperréactivité des voies aériennes induite par l'allergène chez le mammifère test quand ledit mammifère est exposé à l'allergène par comparaison à ce qui est observé en l'absence de l'agent régulateur putatif ;
dans laquelle les agents régulateurs putatifs qui se lient au récepteur du CGRP et réduisent l'hyperréactivité des voies aériennes induite par un allergène chez le mammifère test sont identifiés comme des agents qui réduisent l'hyperréactivité des voies aériennes induite par un allergène.

28. Méthode selon l'une quelconque des revendications 26 à 27, dans laquelle le récepteur du CGRP est un récepteur soluble.

29. Méthode selon l'une quelconque des revendications 26 à 28, dans laquelle à l'étape (a), le récepteur du CGRP est exprimé par une cellule et dans laquelle l'étape de détection (b) comprend également une étape consistant à détecter si le récepteur du CGRP est activé par le composé régulateur putatif.

30. Méthode selon l'une quelconque des revendications 26 à 29, dans laquelle le mammifère test non humain est une souris.

31. Méthode selon l'une quelconque des revendications 26 à 30, dans laquelle l'agent régulateur putatif est le produit d'un processus de conception rationnelle.

32. Méthode selon l'une quelconque des revendications 26 à 31, dans laquelle l'agent régulateur putatif est un anticorps.
